# FASCICULE DE BREVET EUROPEEN

(11) **EP 3 650 035 B1**
(45) Date de publication et mention de la délivrance du brevet: **24.11.2021**
(21) Numéro de dépôt: 19208117.2
(22) Date de dépôt: 08.11.2019
(51) Int. Cl.: A61K 36/04, A61P 1/00, A61P 1/12, A61P 1/04

(54) **EXTRAIT D'ALGUES ROUGES POUR SON UTILISATION POUR LA PREVENTION OU LE TRAITEMENT D'UN TROUBLE INTESTINAL**
ROTALGENEXTRAKT FÜR DEN EINSATZ ZUR VORBEUGUNG UND BEHANDLUNG VON DARMBESCHWERDEN
RED ALGAE EXTRACT FOR USE IN THE PREVENTION OR TREATMENT OF AN INTESTINAL DISORDER

(30) Priorité: 12.11.2018 FR 1860437
(43) Date de publication de la demande: 13.05.2020
(73) Titulaire: OLMIX, 56580 Brehan (FR)
(72) Inventeur: AUBERT, Philippe, 44300 NANTES (FR); BALUSSON, Hervé, 56580 BREHAN (FR); DEMAIS, Hervé, 56700 HENNEBONT (FR); NEUNLIST, Michel, 44000 NANTES (FR); NYVALL-COLLÉN, Pi, 29680 ROSCOFF (FR)
(74) Mandataire: Lavoix

(56) Documents cités:
- WO-A1-2017/132755
- FR-A1- 3 061 013
- W M Sousa ET AL: "Polysaccharide Fraction Extracted from Marine Algae Solieria filiformis Prevents Naproxen-induced Gastrointestinal Damage", , 1 janvier 2016 (2016-01-01), XP055590625, Extrait de l'Internet: URL:http://www.sbbq.org.br/arquivos/2016/c dregional/resumos/R08214-1.pdf [extrait le 2019-05-21]
- SOUSA WILLER M ET AL: "Sulfated polysaccharide fraction from marine algaeSolieria filiformis: Structural characterization, gastroprotective and antioxidant effects", CARBOHYDRATE POLYMERS, APPLIED SCIENCE PUBLISHERS, LTD. BARKING, GB, vol. 152, 29 juin 2016 (2016-06-29), pages 140-148, XP029679009, ISSN: 0144-8617, DOI: 10.1016/J.CARBPOL.2016.06.111
- K. HARDOUIN ET AL: "Biochemical and antiviral activities of enzymatic hydrolysates from different invasive French seaweeds", JOURNAL OF APPLIED PHYCOLOGY., vol. 26, no. 2, 20 novembre 2013 (2013-11-20), pages 1029-1042, XP055590420, NL ISSN: 0921-8971, DOI: 10.1007/s10811-013-0201-6
- BOULHO ROMAIN ET AL: "Antiherpetic (HSV-1) activity of carrageenans from the red seaweedSolieria chordalis(Rhodophyta, Gigartinales) extracted by microwave-assisted extraction (MAE)", JOURNAL OF APPLIED PHYCOLOGY, KLUWER, DORDRECHT, NL, vol. 29, no. 5, 13 juin 2017 (2017-06-13), pages 2219-2228, XP036343053, ISSN: 0921-8971, DOI: 10.1007/S10811-017-1192-5 [extrait le 2017-06-13]

## Description

La présente invention concerne un extrait d'algues rouges de l'espèce *Solieria chordalis* pour son utilisation en tant que complément alimentaire ou pour la prévention et/ou le traitement d'un trouble intestinal, par son effet bénéfique sur la fonction et/ou l'intégrité de la barrière épithéliale intestinale.

Les troubles intestinaux sont de plus en plus répandus de nos jours et regroupent de nombreuses affections touchant aussi bien les personnes jeunes, âgées, les hommes ou les femmes. Le stress, le surmenage, les mauvaises habitudes alimentaires, le vieillissement expliquent en partie l'augmentation de ces troubles.

Les désordres intestinaux sont également très répandus en élevage animal, en particulier en élevage intensif, où, par exemple, les aliments concentrés et hyperprotéiniques qui sont donnés peuvent causer des problèmes digestifs.

Le tube digestif, système complexe, est un organe vital. Il permet à la fois la digestion et l'absorption de nutriments alimentaires mais constitue également la barrière majeure entre l'intérieur de l'organisme et le milieu extérieur. La structure de la barrière intestinale représente une ligne de défense immunologique, physiologique et physique pour l'organisme.

Le stress, le vieillissement, les maladies chroniques sont autant de causes qui fragilisent les fonctions de la barrière intestinale. Son bon fonctionnement permet de maintenir en bonne santé un sujet, d'augmenter sa résistance au stress et de prévenir certaines pathologies chroniques ou infectieuses. Par conséquent, le renforcement de la réponse de l'organisme et en particulier du tube digestif à ce stress représente un enjeu majeur dans le maintien de la santé et pour la prévention ou le ralentissement de l'apparition de pathologies chroniques.

Les algues rouges contiennent des constituants spécifiques (protéines, peptides et polysaccharides, notamment carraghénanes) auxquels ont été associés des effets prébiotiques et de régulation des cellules épithéliales intestinales. Les carraghénanes, et leurs produits de dégradation sont cependant aussi impliqués dans l'induction d'inflammation intestinale, conduisant à des recommandations de limiter l'usage des carraghénanes de poids moléculaire de moins de 50 kDa dans l'alimentation (Cian et al. Marine Drugs, 2015, 13, 5358-5383).

Les inventeurs ont mis en évidence qu'un extrait d'algues rouges du genre Solieria, et notamment deux fractions distinctes de cet extrait, réduisait ou prévenait l'augmentation de la perméabilité intestinale et du temps de transit intestinal, notamment en situation de stress.

### Résumé de l'invention

L'invention est définie dans les revendications.

L'invention concerne un procédé de préparation d'un extrait d'algues rouges de l'espèce *Solieria chordalis* comprenant :
a) le lavage et dessablage des algues;
b) le broyage des algues lavées et dessablées ;
c) l'hydrolyse thermique du broyat obtenu;
d) la séparation de la phase solide du broyat de sa phase liquide ; et
e) la clarification de ladite phase liquide.
f) l'ultrafiltration du jus de filtration obtenu à l'étape e) sur une membrane ayant un seuil de coupure de 300 kDa; et
g) la récolte et le séchage du rétentat d'ultrafiltration obtenu à l'étape f), ou
g) la récolte et le séchage du perméat d'ultrafiltration obtenu à l'étape f).

L'invention concerne également un extrait d'algues rouges de l'espèce *Solieria chordalis* susceptible d'être obtenu par le procédé de préparation selon l'invention.

L'invention concerne également un extrait d'algues rouges de l'espèce *Solieria chordalis* caractérisé en ce que l'extrait d'algues rouges comprend (en poids sur le poids total de l'extrait) :
Matière sèche : 92-99% ;
Matière minérale : 5-37% ;
Sucres : 10-48% ;
Protéines : 10-30% ;
Sulfates : 10-20%.

L'extrait d'algues rouges de l'espèce *Solieria chordalis* est en particulier caractérisé en ce que les monosaccharides constitutifs des polysaccharides de l'extrait d'algues rouges comprennent :
Galactose : 50-75%,
Glucose : 5-40% ; et
Xylose : 1-7%,
les pourcentages étant exprimés en ratio molaire des monosaccharides constitutifs des polysaccharides.

L'extrait d'algues rouges de l'espèce *Solieria chordalis* est en particulier caractérisé en ce que au moins 40% de la matière organique de l'extrait d'algues rouges présente un poids moléculaire supérieur ou égal à 50 kDa, et de préférence au moins 40% de la matière organique de l'extrait d'algues rouges présente un poids moléculaire moyen supérieur ou égal à 250 kDa.

Cet extrait d'algues rouges de l'espèce *Solieria chordalis* est en particulier susceptible d'être obtenu par un procédé selon l'invention dans lequel le rétentat est récolté.

L'invention concerne également un extrait d'algues rouges de l'espèce *Solieria chordalis* caractérisé en ce que l'extrait d'algues rouges comprend (en poids sur le poids total de l'extrait) :
Matière sèche : 95-99% ;
Matière minérale : 5-65%;
Sucres : 5-37% ;
Protéines : 5-29% ;
Sulfates : 1-9%.

L'extrait d'algues rouges de l'espèce *Solieria chordalis* est en particulier caractérisé en ce que les monosaccharides constitutifs des polysaccharides de l'extrait d'algues rouges comprennent :
Galactose : 60-85%,
Glucose : 10-25% ; et
Xylose : 1-5%,
les pourcentages étant exprimés en ratio molaire des monosaccharides constitutifs des polysaccharides.

L'extrait d'algues rouges de l'espèce *Solieria chordalis* est en particulier caractérisé en ce que au moins au moins 70% de la matière organique de l'extrait d'algues rouges présente un poids moléculaire inférieur ou égal à 150 kDa, et de préférence au moins 70% de la matière organique de l'extrait d'algues rouges présente un poids moléculaire moyen inférieur ou égal à 50 kDa.

Cet extrait d'algues rouges de l'espèce *Solieria chordalis* est en particulier susceptible d'être obtenu par un procédé selon l'invention dans lequel le perméat est récolté.

L'invention concerne en outre l'extrait d'algues rouges de l'espèce *Solieria chordalis* selon l'invention pour son utilisation pour la prévention ou le traitement d'un trouble intestinal, caractérisé en ce que l'extrait d'algues rouges de l'espèce *Solieria chordalis* protège et/ou améliore la fonction ou l'intégrité de la barrière épithéliale intestinale et en ce que le trouble intestinal est une diarrhée, une constipation, un syndrôme du côlon irritable, ou une maladie inflammatoire chronique de l'intestin, caractérisé en ce que l'extrait d'algues rouges de l'espèce *Solieria chordalis* réduit l'augmentation de la perméabilité intestinale et/ou du temps de transit intestinal, et le trouble intestinal est induit par un stress.

L'invention concerne aussi un complément alimentaire, une composition pharmaceutique ou un médicament comprenant un extrait d'algues rouges de l'espèce *Solieria chordalis* selon l'invention.

### Description détaillée

### Extrait d'algues rouges

L'extrait d'algues rouges est un extrait d'algues de l'espèce *Solieria chordalis.*

Un dalton (Da) est une unité massique définie comme étant égal à un douzième de la masse d'un atome de carbone 12, masse qui s'avérera ensuite estimée à partir d'un mélange de plusieurs isotopes (principalement carbone 12 et carbone 13, possédant respectivement 6 et 7 neutrons en plus des 6 protons de tout atome de carbone). Un dalton est, avec une assez bonne précision, la masse d'un atome d'hydrogène, la valeur exacte étant 1,00794 uma (unité de masse atomique). Le kilodalton (kDa) est égal à 1000 Da. Dans le cadre de la présente invention, les poids moléculaires mentionnés en kDa sont déterminés par toute méthode usuellement employée par l'homme du métier, en particulier les poids moléculaires des constituants des extraits d'algues pourront être discriminés par ultrafiltration sur des membranes laissant uniquement filtrer des molécules de tailles prédéterminées.

La distribution de poids moléculaires des constituants d'un extrait d'algues peut être déterminée par chromatographie d'exclusion stérique, par exemple sur 2 colonnes Shodex OHpak SB-806M placées en série. L'éluant utilisé est par exemple du nitrate de sodium à 0,1 M, additionné d'azide de sodium à 0,2%, à un débit de 0,5 ml/min. La détection peut être réalisée à l'aide d'un réfractomètre Wyatt et d'un détecteur à diffusion de la lumière 3 angles Wyatt. Dans ces conditions, les valeurs de l'incrément d'indice de réfraction dn/dc seront définies comme égales à 0,142 mL/g.

Selon un mode de réalisation, l'extrait d'algues rouges est caractérisé par les teneurs suivantes (« extrait OSE », susceptible d'être obtenu par le procédé selon l'invention, en tant que rétentat de l'ultrafiltration) :
Matière sèche : 92-99%, de préférence environ 94-99% (en poids sur le poids total de l'extrait) ;
Matière minérale : 20-37%, de préférence 25-32% (en poids sur le poids total de l'extrait) ;
Sucres : 10-38%, de préférences 15-33% (en poids sur le poids total de l'extrait) ;
Protéines : 10-26%, de préférence 13-23% (en poids sur le poids total de l'extrait) ;
Sulfates: 10-15%, de préférence environ 13% (en poids sur le poids total de l'extrait).
L'analyse élémentaire de l'extrait OSE indique que l'extrait est de préférence constitué de : C : 25-30%, de préférence environ 28% ; H : 3-5% ; O : 38-42%, de préférence environ 40% ; N : 1-5%, de préférence environ 3% ; S : 4-8%, de préférence 5-7%.

La composition osidique de la fraction de matière organique de l'extrait OSE (autrement dit, l'extrait déminéralisé) a été déterminée après hydrolyse des polysaccharides par méthanolyse puis triméthylsylilation. Les monosaccharides constitutifs des polysaccharides de l'extrait OSE comprennent ainsi (les pourcentages étant exprimés en ratio molaire des monosaccharides constitutifs des polysaccharides de l'extrait OSE) :
Galactose : 50-75%, de préférence 60-70% ;
Glucose : 5-40% ; et
Xylose : 1-7%.

Selon un mode de réalisation, au moins 40%, de préférence au moins 45%, de la matière organique de l'extrait OSE présente un poids moléculaire supérieur ou égal à 50 kDa.

De préférence, au moins 40%, de préférence au moins 45%, de la matière organique de l'extrait OSE présente un poids moléculaire moyen supérieur ou égal à 250 kDa.

La matière organique d'un extrait d'algues inclut tous les éléments constitutifs de l'extrait, à l'exclusion de la matière minérale, et inclut donc les saccharides (polysaccharides, oligosaccharides) mais également les protéines qui sont associées aux polysaccharides.

Selon un mode de réalisation, l'extrait d'algues rouges est caractérisé par les teneurs suivantes (« extrait OSF », susceptible d'être obtenu par le procédé selon l'invention, en tant que perméat ou filtrat de l'ultrafiltration) :
Matière sèche : 95-99%, de préférence 97-98% (en poids sur le poids total de l'extrait) ;
Matière minérale : 55-65%, de préférence 58-62% (en poids sur le poids total de l'extrait) ;
Sucres : 5-15%, de préférences 7-10% (en poids sur le poids total de l'extrait) ;
Protéines : 5-15%, de préférence 8-10% (en poids sur le poids total de l'extrait) ;
Sulfates : 1-5%, de préférence environ 3% (en poids sur le poids total de l'extrait).

La composition osidique de la fraction de matière organique de l'extrait OSF (autrement dit, l'extrait déminéralisé) a été déterminée après hydrolyse des polysaccharides par méthanolyse puis triméthylsylilation. Les monosaccharides constitutifs des polysaccharides de l'extrait OSF comprennent ainsi (ratio molaire des monosaccharides constitutifs des polysaccharides de l'extrait OSF) :
Galactose : 60-85%, de préférence 70-80% ;
Glucose : 10-25% ; et
Xylose : 1-5%.

Selon un mode de réalisation, au moins 70%, de préférence au moins 80% de la matière organique de l'extrait OSF présente un poids moléculaire inférieur ou égal à 150 kDa.

De préférence au moins 70%, de préférence au moins 80%, de la matière organique de l'extrait OSF présente un poids moléculaire moyen inférieur ou égal à 50 kDa,

L'extrait d'algues rouges est en particulier susceptible d'être obtenu par un procédé de préparation tel que décrit ci-après.

De préférence, l'extrait d'algues rouges réduit ou prévient l'augmentation de la perméabilité intestinale, notamment en situation de stress, et/ou réduit ou prévient l'augmentation du temps de transit intestinal, en particulier induit par le stress. De préférence, l'extrait d'algues rouges induit *in vitro* l'expression de mucines (notamment MUC2 et/ou MUC4) et de protéines de jonction serrées (notamment ZO-1, ZO-2 et/ou claudin-2) dans des cellules intestinales, en particulier des cellules de colon telles que Caco-2 (ATCC® HTB-37).

Selon un mode de réalisation, la perméabilité intestinale est évaluée en mesurant la perméabilité paracellulaire intestinale, par exemple par mesure du passage sanguin d'un marqueur (par exemple acide sulfonique-FITC).

Selon un mode de réalisation, le temps de transit intestinal est évalué par mesure du temps écoulé entre l'ingestion d'un bolus marqué (par exemple avec un colorant) et l'émission de la première fèces contenant le marqueur.

### Procédé d'extraction

Un procédé de préparation de l'extrait d'algues rouges de l'espèce *Solieria chordalis* comprend:
a) le lavage et dessablage d'algues rouges;
b) le broyage des algues lavées et dessablées;
c) l'hydrolyse thermique du broyat obtenu;
d) la séparation de la phase solide du broyat de sa phase liquide ;
e) la clarification de ladite phase liquide;

Le procédé de préparation comprend en outre, après l'étape e) :
f) l'ultrafiltration du jus de filtration obtenu à l'étape e) sur une membrane ayant un seuil de coupure de 300 kDa (et/ou une taille de pores de 0,50 µm) ; et
g) la récolte et le séchage du rétentat d'ultrafiltration obtenu à l'étape f) (« OSE ») ;
   ou,
f) l'ultrafiltration du jus de filtration obtenu à l'étape e) sur une membrane ayant un seuil de coupure de 300 kDa (et/ou une taille de pores de 0,50 µm) ; et
g) la récolte et le séchage du perméat d'ultrafiltration obtenu à l'étape f) (« OSF »).

Selon un mode de réalisation du procédé, les algues sont lavées à l'eau douce.

Elles peuvent être dessablées par tout moyen connu de l'homme du métier.

Lesdites algues sont ensuite broyées, notamment au moyen d'un broyeur, comme par exemple un affineur ou un cutteur. Le broyage conduit de préférence à l'obtention de particules dont la taille est comprise entre 0,1 et 10 mm.

Le broyat est ensuite réhydraté avec de l'eau douce (par exemple avec un ratio en poids broyat : eau de 3 :1 à 1 :1, notamment 2 :1) puis chauffé sous agitation, par exemple pendant 1 à 3h, de préférence pendant environ 2h, à une température de 70 à 85°C, de préférence de 75 à 80°C.

Par la suite, la phase solide du broyat, le marc, est séparée de sa phase liquide, le jus, par pressage du broyat, par exemple à l'aide d'une presse à bande ou à plateaux, ou par centrifugation.

Par « jus », on entend le jus cytoplasmique qui inclut la structure pariétale des cellules des algues.

La phase liquide obtenue est ensuite clarifiée, par exemple avec un clarificateur à assiettes, ou par centrifugation, décantation ou filtration (par exemple à poche ou à plaque).

De préférence, le jus obtenu est ensuite ultrafiltré, après dilution à l'eau douce.

Selon un mode de réalisation du procédé, l'ultrafiltration est réalisée sur une membrane de taille de pores de moins de 1 µm, notamment sur une membrane de taille de pores de 0,8 µm ou de taille de pores de 0,5 µm. De manière préférée, la membrane est une membrane de taille de pores de 0,5 µm. La membrane peut être, par exemple, une membrane de céramique ou une membrane organique. Plus particulièrement, la membrane est une membrane de céramique avec un seuil de coupure placé à 300 kDa, permettant de séparer les polysaccharides de longue chaine (degré de polymérisation > 100) des polysaccharides de plus petite chaine (degré de polymérisation < 100).

Le jus de filtration clarifié obtenu à l'étape e), ou le rétentat ou le perméat d'ultrafiltration, peut ensuite être séché par exemple par lyophilisation ou atomisation.

Optionnellement, l'extrait séché obtenu pourra ensuite être broyé de nouveau, afin d'obtenir une poudre homogène en termes de granulométrie.

Selon un de ces aspects, le procédé se déroule en partie à température ambiante. Par température ambiante, on entend une température comprise entre 5 et 25°C.

Selon un autre de ces aspects, le procédé selon l'invention se déroule en partie à une température comprise entre 4 et 10°C, ceci afin d'éviter les développements microbiens.

Selon un autre de de ces aspects, le procédé selon l'invention se déroule en partie à une température comprise en 70 et 80°C, ceci afin d'améliorer l'extraction.

Ce procédé diffère de la plupart des procédés décrits dans l'art antérieur du fait de l'absence de précipitation de l'extrait d'algues. Il se distingue également des précédents procédés d'extraction par l'absence d'utilisation de solvants, en particulier organiques, ce qui représente un atout majeur du point de vue écologique.

Enfin, selon l'invention le procédé ne met pas en œuvre d'étape de lyse enzymatique.

### Compositions

L'extrait d'algues rouges est de préférence formulé dans une composition qui peut être un complément alimentaire, une composition pharmaceutique ou un médicament.

Un complément alimentaire pour la mise en œuvre de l'invention comprend typiquement l'extrait d'algues rouges et éventuellement au moins un excipient ou additif.

Un complément alimentaire est une denrée alimentaire dont le but est de compléter le régime alimentaire normal et qui constitue une source concentrée de nutriments ou d'autres substances ayant un effet nutritionnel ou physiologique, seuls ou combinés. Un complément alimentaire se présente généralement sous forme de doses, à savoir des formes de présentation telles que les gélules, les pastilles, les comprimés, les pilules et autres formes similaires, ainsi que les sachets de poudre, les ampoules de liquide. Lorsqu'il est destiné à l'alimentation animale, le complément alimentaire peut être mélangé à l'alimentation des animaux, distribué via l'eau de boisson ou administré par des doses orales individuelles.

Une composition pharmaceutique ou médicament pour la mise en œuvre de l'invention comprend typiquement l'extrait d'algues rouges et un excipient pharmaceutiquement acceptable.

Les excipients pharmaceutiquement acceptables utilisés pour la préparation d'un médicament ou d'une composition pharmaceutique comprenant un extrait d'algues rouges pour son utilisation selon l'invention sont choisis selon la forme pharmaceutique et le mode d'administration souhaité, parmi les excipients habituels qui sont connus de l'homme du métier.

Pour une administration orale, sublinguale, sous-cutanée, intramusculaire, intraveineuse, topique, locale, intrapéritonéale, intratrachéale, intranasale, transdermique ou rectale, l'extrait d'algues tel que défini ci-dessus, peut être administré sous forme de doses unitaires, en mélange avec des excipients pharmaceutiques classiques.

Les formes d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, parentérale telle que transdermique, intrapéritonéale, intratrachéale, sous-cutanée, intramusculaire ou intraveineuse, les formes d'administration rectale et les implants.

Lorsqu'une composition solide sous forme de comprimés est préparée, l'ingrédient actif principal peut être mélangé avec un véhicule pharmaceutique, tel que la gélatine, l'amidon, le lactose, le stéarate de magnésium, le talc, la gomme arabique ou analogues.

Les comprimés peuvent également être enrobés avec du saccharose, un dérivé cellulosique, ou d'autres matières appropriées ou encore ils peuvent être traités de telle sorte qu'ils aient une activité prolongée ou retardée et qu'ils libèrent d'une façon continue une quantité prédéterminée de principe actif.

Une préparation sous forme de gélules peut par exemple être obtenue en mélangeant l'ingrédient actif avec un diluant et en versant le mélange obtenu dans des gélules molles ou dures.

Dans un mode de réalisation, l'extrait d'algues rouges, le complément alimentaire, le médicament ou la composition pharmaceutique pour son utilisation selon la présente invention est destiné à une administration orale. Dans un autre mode de réalisation, l'extrait d'algues rouges, le médicament ou la composition pharmaceutique pour son utilisation selon la présente invention est destiné à une administration parentérale.

Les médicaments ou compositions pharmaceutiques comprenant un extrait d'algues rouges pour son utilisation selon l'invention, peuvent aussi se présenter sous forme liquide, par exemple, sous forme de solutions, d'émulsions, de suspensions ou de sirops, et notamment sous une forme adaptée pour une administration orale ou intranasale, par exemple. Les supports liquides appropriés peuvent être, par exemple, de l'eau, des solvants organiques tels que le glycérol ou les glycols, de même que leurs mélanges, dans des proportions variées, dans l'eau.

Une préparation sous forme de sirop ou d'élixir ou pour l'administration sous forme de gouttes peut également contenir l'ingrédient actif conjointement avec un édulcorant, acalorique par exemple, du méthylparabène et du propylparabène comme antiseptique, ainsi qu'un agent donnant du goût et un colorant approprié.

Les poudres ou les granules dispersibles dans l'eau peuvent par exemple contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

En général, dans le cadre de la présente invention, la dose journalière thérapeutique de l'extrait d'algues rouges sera la dose efficace la plus faible de l'extrait d'algues capable de produire un effet prophylactique et/ou thérapeutique sur un trouble intestinal.

Par « dose efficace », on désigne toute quantité d'une composition qui permet d'observer l'effet recherché, ici un effet de protection ou d'amélioration de la fonction ou de l'intégrité de la barrière épithéliale intestinale.

Selon un de ses aspects, un extrait d'algues pour son utilisation selon l'invention est utilisé dans une composition telle que susmentionnée pour une administration à une dose chez l'humain comprise entre 0,1 et 100 mg/kg, encore plus particulièrement entre 0,5 et 60 mg/kg, par exemple entre 1 et 20 mg/kg ou entre 5 et 30 mg/kg, ou à une dose chez l'animal comprise entre 1 et 200 mg/kg, plus particulièrement entre 1 et 100 mg/kg, encore plus particulièrement entre 2 et 45 mg/kg ou entre 10 et 60 mg/kg.

Dans le cas d'un complément alimentaire, destiné à être administré chez un sujet sain, dépourvu de trouble intestinal, la dose journalière de l'extrait d'algues rouges sera une dose contribuant au maintien ou à l'amélioration de la fonction ou de l'intégrité de la barrière épithéliale intestinale.

Par exemple pour l'alimentation animale, le complément alimentaire peut être mélangé à la nourriture des animaux à raison d'une quantité d'extrait d'algues rouge de 0,01 à 10 kg/ tonne de nourriture, par exemple 0,1 à 5 kg/ tonne de nourriture.

### Applications

L'invention concerne l'extrait d'algues rouges de l'espèce *Solieria chordalis* pour son utilisation pour la prévention ou le traitement d'un trouble intestinal caractérisé en ce que l'extrait d'algues rouges protège et/ou améliore la fonction ou l'intégrité de la barrière épithéliale intestinale et en ce que le trouble intestinal est une diarrhée, une constipation, un syndrôme du côlon irritable, ou une maladie inflammatoire chronique de l'intestin, caractérisé en ce que l'extrait d'algues rouges de l'espèce *Solieria chordalis* réduit l'augmentation de la perméabilité intestinale et/ou du temps de transit intestinal, et le trouble intestinal est induit par un stress.

Une méthode de prévention ou traitement d'un trouble intestinal comprenant l'administration, à un sujet qui le nécessite, d'un extrait d'algues rouges de l'espèce *Solieria chordalis* est également proposée.

L'extrait d'algues rouges est en particulier employé sous forme de composition pharmaceutique ou médicament.

Dans le contexte de l'invention, le terme "traiter" ou "traitement" signifie supprimer, alléger ou empêcher la progression d'un désordre ou d'un ou plusieurs symptômes liés à ce désordre. Un traitement "prophylactique" ou "préventif" signifie prévenir l'apparition d'un tel désordre ou d'un ou plusieurs symptômes liés à ce désordre.

L'invention concerne aussi l'extrait d'algues rouges de l'espèce *Solieria chordalis* pour son utilisation non thérapeutique en tant que complément alimentaire, dans l'alimentation humaine ou animale.

Un sujet pour le traitement prophylactique ou thérapeutique, ou l'utilisation en tant que complément alimentaire selon l'invention est un animal, de préférence un mammifère, par exemple un rongeur, un canidé, un félin, un ruminant tel qu'un bovidé (tel qu'un bovin, un caprin, un ovin), un équidé, un porcin, une volaille, un poisson, un crustacé, ou un primate. Un sujet est en particulier un humain, homme, femme ou enfant.

Selon l'invention, l'extrait d'algues rouges protège et/ou améliore la fonction ou l'intégrité de la barrière épithéliale intestinale.

Dans le cas d'une application thérapeutique ou prophylactique, le trouble intestinal est une diarrhée, une constipation, un syndrôme du côlon irritable, ou une maladie inflammatoire chronique de l'intestin, telle que la maladie de Crohn, ou une recto-colite hémorragique.

Le trouble intestinal est un trouble induit par un stress, tel qu'un stress psychologique, infectieux, ou environnemental (c'est-à-dire en lien avec l'exposition du sujet à des molécules par exemple chimiques ou biologiques présentes dans l'environnement de vie du sujet). Selon un mode de réalisation, le stress n'est pas un stress induit par exposition (en particulier par une exposition volontaire) à une molécule chimique ou biologique isolée.

L'extrait d'algues rouges, ou la composition ou le médicament le contenant, est de préférence administré une ou plusieurs fois, si nécessaire, en particulier tant que le sujet présente ou est à risque de développer un trouble intestinal.

Dans la présente demande, le terme "et/ou" est une conjonction grammaticale qui doit être interprétée comme englobant le fait qu'un ou plusieurs des cas qu'elle connecte peuvent se produire. Par exemple, l'expression "du mannose et/ou de l'arabinose " dans l'expression "lesdits polysaccharides comprennent du mannose et/ou de l'arabinose " indique que les polysaccharides peuvent comprendre du mannose, ou de l'arabinose, ou du mannose et de l'arabinose.

Dans l'ensemble de la présente demande, le terme "comprenant" doit être interprété comme englobant toutes les caractéristiques spécifiquement mentionnées, ainsi que des caractéristiques facultatives, additionnelles, non spécifiées. Tel qu'utilisé ici, l'utilisation du terme "comprenant" décrit également le mode de réalisation dans lequel aucune caractéristique autre que les caractéristiques spécifiquement mentionnées n'est présente (c'est-à-dire "consistant en").

La présente invention sera illustrée plus en détail par les figures et exemples ci-dessous qui n'en limitent pas la portée.

### FIGURES

Figure 1 : Protocole d'essai pour l'évaluation des effets *in vitro* de l'extrait d'algue rouges.
Figure 2 : Effet de l'extrait d'algue OSF sur la perméabilité paracellulaire in vivo en situation basale.
Figure 3 : Effet de l'extrait d'algue OSF sur la perméabilité paracellulaire in vivo en situation de stress. Significativité : ¤ : Comparé au témoin.
Figure 4 : Effet de l'extrait d'algue OSF sur la motricité digestive en situation basale.
Figure 5 : Effet de l'extrait d'algue OSF sur la motricité digestive en situation de stress. Significativité :¤ : Comparé au témoin.
Figure 6 : Effet de l'extrait d'algue OSF sur le poids des animaux série 1.
Figure 7 : Effet de l'extrait d'algue OSF sur le poids des animaux série 2.
Figure 8 : Protocole général de l'étude des effets de l'extrait d'algue OSE sur la motricité et la perméabilité intestinale.
Figure 9 : Détails de chaque série d'expériences de l'étude des effets de l'extrait d'algue OSE sur la motricité et la perméabilité intestinale.
Figure 10 : Effet de l'extrait d'algue OSE sur le poids des animaux.
Figure 11 : Effet de l'extrait d'algue OSE sur le poids des cerveaux.
Figure 12 : Effet de l'extrait d'algue OSE sur la perméabilité *in vivo* à l'ASF en situation de stress.
Figure 13 : Effet de l'extrait d'algue OSE sur la perméabilité *ex vivo* du jéjunum à l'ASF en situation de stress.
Figure 14 : Effet de l'extrait d'algue OSE sur la perméabilité *ex vivo* du jéjunum à l'HRP en situation de stress.
Figure 15 : Effet de l'extrait d'algue OSE sur la transition colique en situation de stress.
Figure 16 : Effet de l'extrait d'algue OSE sur taux de corticostérone sanguin en situation de stress.

### EXEMPLES

### Exemple 1 : Préparation d'un extrait d'algue rouge

200 kg d'algues rouge de type *Solieria chordalis,* fraîches, brutes, sont lavées à l'eau douce et dessablées à l'aide d'une machine à laver les algues, ce qui permet d'obtenir environ 150 kg d'algues égouttées à 13% de matière sèche.

Sauf indications contraires, les étapes du procédé sont réalisées à température ambiante.

Les algues sont ensuite broyées en fines particules au moyen d'un affineur industriel (marque Fryma type "ML-180"). Par « fines particules », on entend des particules dont la taille est comprise entre 50 et 3000 nm, avec deux populations, la première dont les tailles sont comprises entre 50 et 200 nm, la seconde dont les tailles sont comprises entre 600 et 3000 nm.

Les algues sont ensuite injectées dans un réacteur avec 0,5 fois leur masse en eau et chauffées pendant 2h, entre 75°C et 80°C, le tout sous agitation (soit une masse totale d'environ 225 kg).

S'en suit le pressage de l'algue au moyen d'une presse à bande industrielle de marque Flottweg type "B FRU 800 HK" à un débit d'environ 1 tonne/heure.

Cette étape permet la séparation de la phase solide (marc) de la phase liquide (jus). Le rendement en jus obtenu est de 55,5%.

Les 110 kg de jus brut obtenus sont ensuite filtrés au moyen d'un filtre à plaque sur des plaques de taille de pores de 9 µm.

On obtient ainsi 100 kg d'un jus clair à 3,50 % de matière sèche (90% de rendement en masse) et environ 10 kg d'une crème à 10,1% de matière sèche (10% de rendement en masse).

Un ajout d'eau douce dans le jus clair est effectué représentant 0,5 fois la masse du jus (50 kg).

Par la suite, ce jus dilué est ultrafiltré sur une membrane céramique (Pall) de taille de pores de 0,5 µm. Cette membrane présente un seuil de coupure de 300 kDa.

On obtient ainsi un perméat et un rétentat. Le rétentat est concentré jusqu'à l'obtention de 15 kg final (10% du volume de départ) à 7,7% de matière sèche.

On obtient donc deux fractions d'ultrafiltration du jus de l'algue *Solieria chordalis :*
- une fraction « OSF » constituée du perméat ou filtrat;
- une fraction « OSE » constituée du rétentat.

Le rétentat et le filtrat sont alors séchés par lyophilisation.

La lyophilisation est réalisée à une température de congélation de -80°C qui est également la température minimale au cours de cette étape.

La poudre obtenue est ensuite broyée avec un broyeur planétaire MiniMill de marque Philips. Le produit a été introduit dans des bols de broyage (10 g de produit dans chaque bol de broyage avec 4 billes en zircon). L'ensemble a été mis en rotation pendant 15 minutes à la vitesse 10.

### Exemple 2 : Caractérisation des effets in vitro de l'extrait d'algues

### MATERIELS ET METHODES

### Cultures cellulaires

Les cellules Caco-2 sont des cellules de type entérocytaire provenant du carcinome colorectal humain (ATCC® HTB-37™, Molsheim, France) et les cellules HT29-MTX, une lignée cellulaire caliciforme sécrétant de la mucine dérivée de carcinome du colon humain, ont été fournies par T. Lesuffleur (INSERM U560, Lille, France). Les deux lignées cellulaires ont été cultivées séparément dans du Milieu d'Eagle Modifié (DMEM) de Dulbecco avec une teneur élevée en glucose (4,5 g/L) contenant 10% de sérum fœtal de veau (DMEM 10% SVF, Laboratoires PAA, Les Mureaux, France) et des antibiotiques (streptomycine 100 µg/mL et pénicilline 100 unités mL, Sigma Aldrich, St. Quentin Fallavier, France). La différenciation a été achevée 15 jours après la confluence pour les cellules Caco-2 et à 21 jours pour les cellules HT29-MTX. Les cellules ont été incubées à 37°C dans 5% de CO₂ et 95% d'humidité relative.

Pour les conditions basales, l'extrait d'algues a été ajouté au milieu de culture cellulaire pendant 1 heure ou 4 heures de stimulation, sur les cellules Caco-2 à 15 jours après la confluence, et à 21 jours après la confluence pour les cellules HT29-MTX. Les cellules, après 1 h ou 4 h de stimulation, ont ensuite été abondamment lavées et récoltées pour l'extraction de l'ARNm.

Pour les conditions «inflammatoires», une pré-stimulation des cellules (cellules Caco-2 à 15 jours après la confluence et à 21 jours post-confluence pour les cellules HT29-MTX) par LPS à 10 ng/mL a été réalisée pendant 3 heures. L'extrait a ensuite été ajouté au milieu de culture contenant le LPS pendant 1h ou 4h de plus. Les cellules, après 1 h ou 4 h de stimulation, ont ensuite été abondamment lavées et récoltées pour l'extraction de l'ARNm.

### Evaluation de l'expression des gènes par qRT-PCR

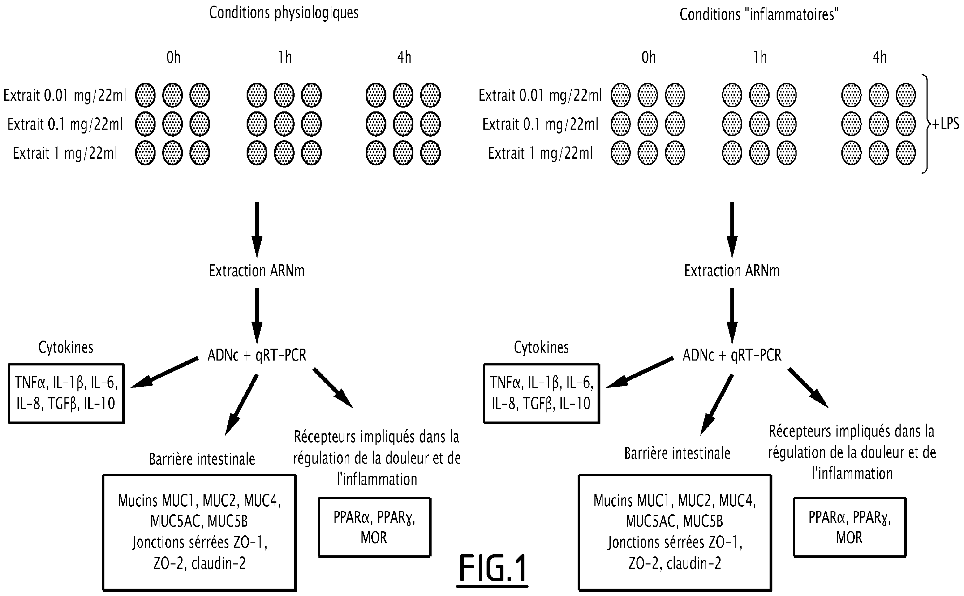

L'expression des gènes GAPDH, TNFα, IL-1β, IL-6, IL-8, IL-10, TGFβ, PPARα, PPARγ, MOR, ZO-1, ZO-2, Claudin2, MUC1, MUC2, MUC4, MUC5AC, et MUC5B a été quantifiée comme suit (voir Figure 1).

L'ARNm total a été isolé des cellules en utilisant le kit Rneasy (Macherey Nagel, Hoerdt, France) selon les instructions du fabricant. La quantification de l'ARN a été réalisée par spectrophotométrie. Après traitement à 30°C pendant 30 minutes avec 20-50 unités de DNase I exempte de RNase (Roche Diagnostics Corporation, Indianapolis, IN, USA), des amorces oligo-dT (Roche Diagnostics Corporation, Indianapolis, USA) et le kit de transcription inverse de l'ADNc de haute capacité Applied Biosystems) ont été utilisés pour synthétiser l'ADNc simple brin. L'ARNm a été quantifié à l'aide de SYBR green Master Mix (Applera, Courtaboeuf, France) avec des oligonucléotides spécifiques de souris dans un GeneAmp Abiprism 7000 (Applera, Courtaboeuf, France). Dans chaque essai, des contrôles calibrés et sans échantillon ont été inclus. Chaque échantillon a été analysé en triple. L'intensité du colorant vert SYBR a été analysée à l'aide du logiciel Abiprism 7000 SDS (Applera, Courtaboeuf, France). Tous les résultats ont été normalisés par rapport au gène GAPDH.

Tous les résultats sont exprimés en tant que multiple de la moyenne d'activation du triplicata / moyenne du triplicata dans le milieu seul.

Les résultats sont considérés comme significatifs dès lors qu'une augmentation ou réduction de l'expression d'un gène d'un facteur 2, au moins, est observées par rapport au niveau observés dans le milieu de culture seul.

### RESULTATS

### Effet de la stimulation des cellules par l'extrait d'algues OSE sur l'expression des cytokines

### i. Dans les conditions basales

L'effet de l'extrait OSE a été évalué sur l'expression de cytokines pro-inflammatoires (IL-1β, TNFα, IL-6 IL-8 et TGFβ) et sur I'IL-10, une cytokine anti-inflammatoire.

Dans les cellules Caco-2, une diminution du TNFα a été observée avec l'extrait à 0,1 et 0,01 mg/mL après 1 heure de stimulation. Cet effet a été maintenu après 4h de stimulation. Une diminution du taux d'ARNm du TGFβ at a également été observée après 1 et 4 h. Après 4h de stimulation avec l'extrait, une diminution de l'expression de I'IL-6 est enregistrée. L'extrait à 0,1 mg/mL induit également l'expression d'IL-10 (augmentation de 6,98 fois).

En conditions basales, la stimulation des cellules Caco-2 par l'extrait est sûre aux trois concentrations testées (0,01, 0,1 et 1 mg/mL) et diminue l'expression des cytokines pro-inflammatoires (TNFα, TGF-β et IL-6) et augmente l'expression de I'IL-10, une cytokine anti-inflammatoire.

Dans les cellules HT29-MTX, un profil d'expression génique différent après stimulation par l'extrait a été observé, montrant une augmentation de l'expression d'IL-6 après 1h de stimulation (augmentation de 2,25 fois par rapport au milieu seul pour l'extrait à 0,01 mg/mL, et d'un facteur 2,45 avec l'extrait à 0,1 mg/mL). L'augmentation de l'expression d'IL-6 a également été observée après 4 h de stimulation avec un niveau d'augmentation similaire (2,29 pour l'extrait à 0,01 mg/mL et 3,03 à 1 mg/mL).

Une augmentation de l'expression des gènes IL-1β et IL-8 a été enregistrée après 4h pour les cellules HT29-MTX avec l'extrait à 0,1 et 1 mg/mL, atteignant une augmentation de l'expression génique d'un facteur environ 18 avec la concentration plus élevée de l'extrait pour ces deux gènes.

L'effet pro-inflammatoire faible de l'extrait dans les cellules HT29-MTX, dans les conditions basales, peut jouer un rôle pour permettre de renforcer la barrière intestinale et les défenses.

### ii. Dans les conditions « inflammatoires »

L'effet de l'extrait OSE a été évalué sur l'expression de cytokine pro-inflammatoire (IL-1β, TNF-α, IL-6, IL-8 et TGF-β) et de I'IL-10, une cytokine anti-inflammatoire, dans les cellules stimulées par le LPS à 10ng/mL pour induire des conditions inflammatoires.

Dans les cellules Caco-2 dans des conditions inflammatoires, un profil d'expression génique similaire a été observé après 1h ou 4h de stimulation des cellules inflammatoires avec l'extrait, montrant une augmentation précoce et transitoire de l'expression du TNFα suivie d'une forte diminution de l'expression du TNF-α après 4h de stimulation. Un effet similaire a été observé pour IL-8, IL-10 et TGF-β.

Une augmentation de l'expression de I'IL-1β et de I'IL-6 au niveau de I'ARNm a été observée dans les cellules Caco-2 "inflammatoires".

### L'extrait ioue un rôle immunomodulateur dans les cellules Caco-2 en cas d'inflammation

Dans les cellules HT29-MTX « inflammées », seule une augmentation de l'expression des gènes de cytokine a été enregistrée, avec un effet observé essentiellement après 4 heures de stimulation. L'effet de la stimulation par l'extrait OSE est observé avec les 3 concentrations testées (0,01, 0,1 et 1 mg/mL). L'extrait semble induire une réponse pro-inflammatoire dans les conditions inflammatoires. En effet, même si l'augmentation de l'expression des gènes IL-1β et IL-6 reste modérée, l'extrait induit une forte augmentation du TNF-α (augmentation d'un facteur 38,19 avec l'extrait à 1 mg/mL), de I'IL-8 (58,81 fois plus), et du TGF- β (27,19 fois plus).

**Tableau 1 : stimulation de l'expression de gènes codant pour des cytokines**

| | | **Condition basale** | | | | | | | **Condition inflammatoire** | | | | | | |
|---|---|---|---|---|---|---|---|---|---|---|---|---|---|---|---|
| | | | **IL-1β** | **TNFα** | **IL-6** | **IL-8** | **TGFβ** | **IL-10** | | **IL-1β** | **TNFα** | **IL-6** | **IL-8** | **TGFβ** | **IL-10** |
| **cellules Caco-2** | Medium | 1H | 1,00 | 1,00 | 1,00 | 1,00 | 1,00 | 1,00 | LPS 1H | 1,00 | 1,00 | 1,00 | 1,00 | 1,00 | 1,00 |
| | E1-0,01 | | 2,02 | 0,01 | 1,21 | 0,69 | 0,34 | 0,68 | | 1,59 | 0,99 | 1,14 | 1,42 | 0,84 | 0,96 |
| | E1-0,1 | | 1,59 | 0,01 | 1,72 | 0,50 | 0,33 | 0,35 | | 1,00 | 16,06 | 0,90 | 1,07 | 0,57 | 3,81 |
| | E1-1 | | 0,96 | 2,70 | 1,23 | 1,22 | 0,72 | 1,20 | | 0,89 | 1,83 | 1,20 | 8,42 | 3,92 | 1,95 |
| | Medium | 4H | 1,00 | 1,00 | 1,00 | 1,00 | 1,00 | 1,00 | LPS 4H | 1,00 | 1,00 | 1,00 | 1,00 | 1,00 | 1,00 |
| | E1-0,01 | | 1,80 | 0,07 | 0,56 | 0,93 | 0,18 | 0,55 | | 5,82 | 0,00 | 5,70 | 0,24 | 0,31 | 0,00 |
| | E1-0,1 | | 0,35 | 0,81 | 0,17 | 0,41 | 0,37 | 6,98 | | 7,94 | 0,01 | 13,01 | 0,32 | 0,15 | 0,01 |
| | E1-1 | | 0,73 | 0,05 | 0,35 | 1,09 | 0,91 | 1,44 | | 7,37 | 0,01 | 5,38 | 0,26 | 0,46 | 0,00 |
| **cellules HT-29MTX** | Medium | 1H | 1,00 | 1,00 | 1,00 | 1,00 | 1,00 | 1,00 | LPS 1H | 1,00 | 1,00 | 1,00 | 1,00 | 1,00 | 1,00 |
| | E1-0,01 | | 1,20 | 0,68 | 2,25 | 2,12 | 1,01 | 1,58 | | 1,02 | 0,89 | 0,92 | 1,43 | 0,65 | 0,72 |
| | E1-0,1 | | 0,93 | 1,11 | 2,44 | 1,44 | 1,36 | 0,55 | | 1,00 | 1,00 | 0,45 | 1,66 | 0,77 | 0,47 |
| | E1-1 | | 1,05 | 1,53 | 0,98 | 1,99 | 1,58 | 1,52 | | 2,11 | 2,08 | 0,76 | 3,47 | 0,78 | 1,90 |
| | Medium | 4H | 1,00 | 1,00 | 1,00 | 1,00 | 1,00 | 1,00 | LPS 4H | 1,00 | 1,00 | 1,00 | 1,00 | 1,00 | 1,00 |
| | E1-0,01 | | 1,70 | 0,69 | 2,29 | 1,64 | 1,14 | 0,56 | | 3,24 | 4,24 | 2,04 | 58,81 | 27,19 | 2,28 |
| | E1-0,1 | | 2,59 | 0,96 | 0,53 | 3,95 | 1,24 | 1,78 | | 2,40 | 2,36 | 1,46 | 2,78 | 1,41 | 1,43 |
| | E1-1 | | 17,80 | 1,14 | 3,02 | 18,72 | 1,06 | 1,22 | | 5,77 | 38,19 | 13,52 | 34,23 | 2,19 | 14,11 |

OSE produit un effet immunomodulateur intéressant sur les cellules Caco-2 et HT-29 MTX avec un effet plus marqué dans les conditions inflammatoires. Cette propriété immunomodulatrice d'OSE pourrait jouer un rôle dans le renforcement de la barrière intestinale. De plus, la stimulation des cellules épithéliales du colon par OSE n'est pas délétère dans les conditions basales ou inflammatoires.

### Effets de OSE sur les récepteurs impliqués dans l'inflammation et l'expression de la régulation de la douleur

### i. Dans les conditions basales

Les effets de l'extrait OSE ont été évalués sur l'expression de récepteurs jouant un rôle régulateur sur l'inflammation (PPARγ et MOR) et sur la douleur viscérale (MOR et PPARα).

Sur les cellules Caco-2, un effet significatif et très important induit par l'extrait OSE a été observé seulement sur l'expression de MOR dans les cellules stimulées par la concentration la plus élevée d'OSE à 1 mg/ml après 1h de stimulation.

Dans les cellules HT-29 MTX, une hausse (facteur 2.57) de PPARγ a été enregistrée dans les cellules stimulées par OSE à la concentration la plus élevée (1 mg/ml) et une hausse (facteur 2,32) a été observée pour MOR (E1 concentré à 0,01 mg/ml) après 1h de stimulation.

Après 4h de stimulation par OSE à 0,1 et 1 mg/ml, une augmentation soutenue de l'expression du gène MOR a été observée avec une hausse respective d'un facteur 2,25 et 2,91-par rapport au milieu seul.

OSE induit une hausse soutenue après 4h de stimulation cellulaire de l'expression de MOR dans HT-29MTX. L'effet sur la hausse de l'expression de MOR dans l'expression basale était seulement transitoire sur les cellules Caco-2.

### ii. Dans des conditions inflammatoires

Les effets de l'extrait OSE ont été évalués sur l'expression des récepteurs régulateurs de l'inflammation (PPARγ and MOR) et sur la douleur viscérale (MOR et PPAPγ) sur les cellules pré-stimulées par LPS (état inflammatoire).

Sur les cellules Caco-2, OSE concentré à 0,1 et 1 mg/ml induit une hausse précoce de l'expression de MOR, respectivement une hausse facteur 4 et 2,56 par rapport au milieu seul après 1h de stimulation

Après 4h de stimulation des cellules Caco-2 enflammées, une très forte hausse de l'expression du gène PPARγ a été observée (facteur de 60,76). De façon surprenante, l'expression du gène MOR a totalement été inhibée après 4h de stimulation cellulaire par OSE pour les trois concentrations testées.

Pour HT-29MTX pré-stimulé par LPS, aucun effet de l'extrait OSE aux différentes concentrations n'a été observé concernant l'expression du gène de MOR, PPARα ou PPAPγ après 1h de stimulation.

Après 4h de stimulation par OSE au dosage le plus élevé de 1 mg/ml, une hausse importante de l'expression de PPARγ et MOR a été observée correspondant respectivement à une induction facteur 20,70 et 11,55 comparé au milieu seul. L'expression de PPARα a également été induite par OSE à 0,01 et 0,1 mg/ml avec une hausse respective facteur 7,6 et 2,61 de l'expression du gène au niveau de l'ARNm.

**Tableau 2 : Stimulation de l'expression de gènes codant pour des récepteurs impliqués dans la régulation de l'inflammation et de la douleur**

| | | **Condition basale** | | | | **Condition inflammatoire** | | | |
|---|---|---|---|---|---|---|---|---|---|
| | | | **PPAPγ** | **MOR** | **PPARα** | | **PPAPγ** | **MOR** | **PPARα** |
| **cellules Caco-2** | Médium | 1H | 1,00 | 1,00 | 1,00 | LPS 1H | 1,00 | 1,00 | 1,00 |
| | E1-0,01 | | 1,91 | 0,56 | 1,20 | | 1,06 | 1,12 | 0,70 |
| | E1-0,1 | | 1,68 | 0,21 | 1,35 | | 0,43 | 4,00 | 0,25 |
| | E1-1 | | 0,43 | 400,00 | 1,12 | | 0,71 | 2,56 | 0,62 |
| | Medium | 4H | 1,00 | 1,00 | 1,00 | LPS 4H | 1,00 | 1,00 | 1,00 |
| | E1-0,01 | | 0,69 | 0,00 | 0,17 | | 22,31 | 0,00 | 51,91 |
| | E1-0,1 | | 0,34 | 0,52 | 0,36 | | 15,54 | 0,01 | 4,40 |
| | E1-1 | | 1,33 | 0,01 | 1,55 | | 26,55 | 0,00 | 60,76 |
| **cellules HT-29MTX** | Médium | 1H | 1,00 | 1,00 | 1,00 | LPS 1H | 1,00 | 1,00 | 1,00 |
| | E1-0,01 | | 1,70 | 2,32 | 0,64 | | 0,57 | 1,11 | 0,59 |
| | E1-0,1 | | 1,47 | 0,46 | 0,53 | | 0,75 | 0,44 | 0,57 |
| | E1-1 | | 2,57 | 1,50 | 0,79 | | 0,71 | 1,86 | 0,65 |
| | Médium | 4H | 1,00 | 1,00 | 1,00 | LPS 4H | 1,00 | 1,00 | 1,00 |
| | E1-0,01 | | 1,07 | 0,47 | 1,77 | | 0,93 | 1,66 | 7,60 |
| | E1-0,1 | | 0,88 | 2,25 | 1,99 | | 1,92 | 0,98 | 2,61 |
| | E1-1 | | 0,83 | 2,91 | 1,41 | | 2,70 | 11,55 | 1,18 |

En condition inflammatoire, OSE induit une hausse importante des récepteurs MOR et PPARα au niveau des cellules Caco-2 et HT-29 MTX. MOR est nettement impliqué dans la régulation de l'inflammation et l'analgésie au niveau du colon et la hausse de l'expression de ce récepteur peut être impliquée au moins en partie par l'effet anti-inflammatoire et immunomudulateur d'OSE. Concernant, PPARα, ce récepteur est connu pour être impliqué dans la régulation de la douleur intestinale.

### Effets de OSE sur la barrière intestinale

Les effets de l'extrait d'OSE ont été évalués sur l'expression des mucines (MUC1, MUC2, MUC4, MUC5AC et MUC5B) et des protéines de jonctions serrées (ZO-1 ; ZO-2 et Claudin-2) afin d'évaluer si l'extrait OSE peut favoriser un effet bénéfique sur le renforcement de la barrière intestinale.

### i. Effet sur les mucines en conditions basales

Dans les cellules Caco-2, OSE provoque une induction précoce de l'expression du gène de la mucine. En effet, l'augmentation de l'expression du gène MUC2 a été observée pendant 1 heure après la stimulation par OSE aux trois concentrations testées et cet effet a été maintenu après 4h de stimulation. Des résultats similaires ont été observés concernant l'expression génique de MUC4. Une induction transitoire de MUC5B avec la concentration la plus élevée de OSE a également été observée.

Ces résultats ont été confirmés au niveau des cellules MTX HT-29 mucosécrétrices montrant que l'extrait OSE induisait l'expression de MUC2 et MUC4 avec un effet plus important après 4h de stimulation.

### ii. Effet sur les mucines en condition inflammatoire

Sur les cellules Caco-2 « enflammées », le profil d'expression génique induit par l'extrait OSE est différent. Une hausse de MUC4 seulement a été observée après 1h de stimulation par l'extrait OSE pour les trois concentrations testées. Après 4h de stimulation, une hausse de l'expression du gène MUC5AC a été observée également avec les trois concentrations de OSE testée.

Au niveau des cellules "enflammées" HT-29MTX, l'induction du gène MUC2 au niveau de I'ARNm pour les trois concentrations testées de l'extrait OSE a été observée après 4h de stimulation cellulaire.

L'extrait OSE induit une hausse important de l'expression des gènes MUC2 et MUC4 en condition basale et inflammatoire. Cette propriété de OSE pourrait un effet très intéressante sur le renforcement de la barrière intestinale.

### iii. Effet sur les protéines de jonctions serrées en condition basale

Au niveau des cellules Caco-2, OSE, à la concentration la plus élevée de 1mg/ml induit l'expression de claudin-2 par un facteur 6,58-ainsi que l'expression de ZO-1 (facteur 3,30- par rapport aux cellules de contrôle).

Au niveau des cellules HT-29 MTX, l'induction de claudin-2 par OSE à 1mg/ml après une heure de stimulation cellulaire a été confirmée (induction de 2,69-fois par rapport aux cellules de contrôle).

En condition basale, un effet très limité de la stimulation par OSE sur les protéines de jonction serrées a été observé au niveau des cellules HT-29 MTX ou Caco-2.

### iv. Effet sur les protéines de jonction serrées en condition inflammatoire

Sur les cellules Caco-2 « enflammées », OSE, à une concentration de 0,1 et 1 mg/ml, induit une expression précoce importante de ZO-2, respectivement facteur 24,49 et 12,93 en comparaison avec les cellules de contrôle, après une heure de stimulation. Puis, après 4 heures de stimulation cellulaire, un niveau élevé d'expression génique de ZO-1 a été observé avec les trois concentrations testées de l'extrait OSE avec une induction respective facteur 46,85, 28,52 et 44,41 de l'expression de ZO-1.

Au niveau des cellules HT-29 MTX, l'induction de la hausse de l'expression du gène ZO-1 a également été observée après 4 heures de stimulation ainsi que la hausse de l'expression de ZO-2 avec la concentration la plus élevée d'OSE. L'expression de Claudin-2 a également été augmentée avec OSE à 1 mg/ml après 1 heure de stimulation cellulaire.

En condition inflammatoire, l'extrait OSE induit la hausse de l'expression des protéines de jonction serrées au niveau des cellules HT-29 MTX et Caco-2 en faveur d'un rôle intéressant de l'extrait E1 sur le renforcement de la barrière cellulaire.

**Tableau 3 : Stimulation de la barrière intestinale : expression de gènes codant pour les mucines et des protéines de jonctions serrées**

| | | | Condition basale | | | | | | |
|---|---|---|---|---|---|---|---|---|---|
| | | | MUC2 | MUC4 | MUC5AC | MUC5B | ZO-1 | ZO-2 | Claudin2 |
| cellules Caco-2 | Medium | 1H | 1,00 | 1,00 | 1,00 | 1,00 | 1,00 | 1,00 | 1,00 |
| | E1-0,01 | | 2,27 | 1,48 | 1,14 | 1,50 | 1,63 | 0,02 | 0,93 |
| | E1-0,1 | | 2,11 | 2,11 | 0,81 | 0,96 | 1,38 | 0,02 | 1,01 |
| | E1-1 | | 43,04 | 435,61 | 0,67 | 7,82 | 0,47 | 0,74 | 6,58 |
| | Medium | 4H | 1,00 | 1,00 | 1,00 | 1,00 | 1,00 | 1,00 | 1,00 |
| | E1-0,01 | | 1,85 | 1,30 | 0,37 | 0,21 | 1,88 | 0,54 | 0,87 |
| | E1-0,1 | | 5,85 | 8,23 | 0,43 | 0,43 | 1,89 | 0,36 | 0,92 |
| | E1-1 | | 2,62 | 4,52 | 0,95 | 0,57 | 3,30 | 0,31 | 0,26 |
| cellules HT-29MTX | Medium | 1H | 1,00 | 1,00 | 1,00 | 1,00 | 1,00 | 1,00 | 1,00 |
| | E1-0,01 | | 2,28 | 0,85 | 0,76 | 0,98 | 0,78 | 0,85 | 1,56 |
| | E1-0,1 | | 0,87 | 0,42 | 1,27 | 0,79 | 0,73 | 0,74 | 1,22 |
| | E1-1 | | 1,36 | 2,35 | 1,54 | 1,26 | 0,85 | 1,10 | 2,69 |
| | Medium | 4H | 1,00 | 1,00 | 1,00 | 1,00 | 1,00 | 1,00 | 1,00 |
| | E1-0,01 | | 3,20 | 0,33 | 0,75 | 0,62 | 0,82 | 0,63 | 0,95 |
| | E1-0,1 | | 2,62 | 2,52 | 1,03 | 1,40 | 1,48 | 0,35 | 1,54 |
| | E1-1 | | 3,39 | 3,41 | 1,18 | 1,80 | 1,50 | 1,33 | 1,07 |

| | | | Condition inflammatoire | | | | | | |
|---|---|---|---|---|---|---|---|---|---|
| | | | MUC2 | MUC4 | MUC5AC | MUC5B | ZO-1 | ZO-2 | Claudin2 |
| cellules Caco-2 | Medium | LPS 1H | 1,00 | 1,00 | 1,00 | 1,00 | 1,00 | 1,00 | 1,00 |
| | E1-0,01 | | 1,80 | 3,04 | 0,98 | 1,20 | 1,44 | 1,36 | 0,87 |
| | E1-0,1 | | 1,86 | 2,38 | 0,37 | 1,37 | 0,56 | 24,49 | 0,86 |
| | E1-1 | | 1,55 | 2,57 | 1,49 | 1,22 | 1,11 | 12,93 | 1,50 |
| | Medium | LPS 4H | 1,00 | 1,00 | 1,00 | 1,00 | 1,00 | 1,00 | 1,00 |
| | E1-0,01 | | 0,12 | 0,01 | 4,77 | 0,39 | 46,85 | 0,05 | 0,18 |
| | E1-0,1 | | 0,04 | 0,01 | 2,09 | 0,19 | 28,52 | 0,07 | 0,07 |
| | E1-1 | | 0,10 | 0,02 | 5,42 | 0,34 | 44,41 | 0,06 | 0,14 |
| cellules HT-29MTX | Medium | LPS 1H | 1,00 | 1,00 | 1,00 | 1,00 | 1,00 | 1,00 | 1,00 |
| | E1-0,01 | | 0,54 | 1,63 | 0,76 | 1,46 | 0,39 | 0,43 | 0,99 |
| | E1-0,1 | | 0,51 | 0,34 | 0,37 | 0,67 | 0,71 | 0,37 | 1,00 |
| | E1-1 | | 0,89 | 0,26 | 0,90 | 2,31 | 0,57 | 0,31 | 2,82 |
| | Medium | LPS 4H | 1,00 | 1,00 | 1,00 | 1,00 | 1,00 | 1,00 | 1,00 |
| | E1-0,01 | | 2,92 | 0,69 | 1,37 | 0,63 | 1,76 | 0,95 | 0,45 |
| | E1-0,1 | | 3,79 | 1,34 | 1,22 | 0,75 | 2,63 | 1,77 | 1,05 |
| | E1-1 | | 2,81 | 0,73 | 0,83 | 0,73 | 5,58 | 2,26 | 1,80 |

### Résumé des effets de OSE:

- L'extrait OSE possède des propriétés immunomodulatrices modérées in vitro sur les cellules Caco-2 et HT-29 MTX en condition basale et en condition inflammatoire (propriétés anti-inflammatoires sur les cellules Caco-2 et propriétés pro-inflammatoires modérées sur les cellules HT-29 MTX) ;
- L'extrait OSE est capable d'induire l'expression des récepteurs MOR, PPARα et PPARγ. Ces récepteurs jouant un rôle dans la régulation de l'inflammation et de la douleur au niveau du colon ;
- OSE induit l'expression des mucines (MUC2, MUC4) et des protéines de jonction serrées. Ces composés jouant un rôle dans l'intégrité et le renforcement de la barrière muqueuse.

### Exemple 3: Etude préliminaire des effets d'extraits d'algues sur la motricité et la perméabilité intestinale

L'impact de deux extraits d'algues rouges, OSE et OSF (extrait d'algues rouges *Solieria chordalis* (perméat ou filtrat)), sur des fonctions clefs du tube digestif, *i.e.* la fonction de barrière intestinale et la motricité intestinale, a été évalué. Cette analyse visait dans un premier temps à déterminer la capacité des extraits d'algues à modifier ces fonctions digestives à la fois à leur état de base et dans un modèle de stress (Stress d'évitement de l'eau) connu pour les modifier, en partie via l'activation de l'axe hypothalamo-hypophyso-surrénalien (axe HPA). L'impact de l'extrait d'algues (administré pendant 14 jours) a été évalué sur la motricité et la perméabilité intestinale *in vivo* chez la souris adulte maintenue dans des conditions normales puis dans des conditions de stress. L'impact de ce régime sur les modifications des fonctions intestinales était ainsi évalué avant le début du traitement (JO), après 10 jours de traitements (J10) et enfin après 4 jours de stress (modèle WAS : Water Avoidance Stress - ou Stress d'évitement de l'eau) (J11 à J14). A la fin de cette phase de stress, l'impact de l'extrait d'algues sur la réponse au stress était également évalué en mesurant le taux sanguin de corticostérone (hormone du stress).

Les mêmes expériences ont été réalisées sur six extraits d'algues (OSA : extrait d'algues vertes *Ulva* sp., OSB : extrait d'algues vertes *Ulva* sp., OSC : extrait d'algues vertes *Ulva* sp., OSD : extrait d'algues vertes *Ulva* sp., OSE : extrait d'algue rouge *Solieria chordalis (retentât)* et OSF : extrait d'algues rouges *Solieria chordalis* (perméat ou filtrat), préparés selon le même procédé que décrit dans l'exemple 1.

### MATERIELS ET METHODES

### Modèle animal

Le modèle animal utilisé était la souris saine C57 black 6Rj âgée de 8 semaines. 5 jours après leur arrivée (période d'acclimatation), les souris étaient gavées quotidiennement durant 14 jours avec l'extrait d'algues ou bien un placebo (excipient). A partir du onzième jour de traitement, les souris étaient placées durant 1 heure sur un plot entouré d'eau durant 4 jours consécutifs jusqu'au quatorzième jour de traitement.

Les souris étaient hébergées dans des cages sur portoir ventilé. L'extrait d'algues et le placebo étaient testés successivement sur 2 groupes de 6 souris, soit un total de 12 animaux par condition. Le poids des animaux était mesuré à J1, J8 et J13.

### Evaluation des effets des oligosaccharides sur la perméabilité et la motricité intestinale chez la souris

### Etude de la perméabilité para cellulaire in vivo :

La perméabilité paracellulaire intestinale était mesurée *in vivo* avant le début des gavages (J0) puis après 10 jours et 14 jours de gavage en mesurant le passage sanguin d'un marqueur spécifique (acide sulfonique-FITC, 400Da).

### Etude de la motricité gastro-intestinale in vivo :

La motricité digestive était évaluée *in vivo* en mesurant le temps de transit total et en évaluant le transit colique avant le début des gavages (J0) puis après 10 jours et 14 jours de traitement.

Mesure du temps de transit total : La souris était gavée avec une solution de carboxy-méthyl-cellulose contenant du rouge carmin. Le temps de transit total correspondait au temps écoulé entre le gavage et l'émission de la première fèces rouge. Il n'était pas évalué à J14.

Evaluation du transit colique : Chaque souris était placée en cage individuelle. Les fèces émises étaient relevées et comptabilisées au bout d'une heure puis 2 heures. Les fèces étaient ensuite pesées (poids frais) puis séchées et enfin repesées (poids sec) afin de déterminer le pourcentage d'humidité.

### Etude de l'impact du complément alimentaire sur la réponse au stress :

L'impact du complément alimentaire sur le stress éprouvé par l'animal était évalué en mesurant le taux de corticostérone lors du stress d'évitement de l'eau. La concentration sanguine en corticostérone était déterminée par dosage ELISA.

### Prélèvement et stockage d'échantillons

Au sacrifice, des segments de jéjunum, d'iléon, de côlon proximal et de côlon distal étaient d'une part prélevés et congelés (-80°C) dans des tubes secs, et d'autre part, fixés au PBS/paraformaldéhyde 4%. Le cerveau était également prélevé, la moitié était congelé à -80°C et l'autre était fixée. Ces échantillons pourront servir respectivement à d'éventuelles analyses protéomiques (tubes secs) et des analyses histologiques (tissus fixés). Des fèces étaient également prélevées et congelées à -80°C pour des études ultérieures éventuelles sur le microbiote.

### RESULTATS

### Effet de l'extrait d'algues OSE sur la perméabilité paracellulaire in vivo

Le détail des résultats obtenus avec l'extrait OSE est décrit dans l'exemple 4 suivant, qui est la synthèse de deux études indépendantes de caractérisation des effets de l'extrait OSE.

### Effet de l'extrait d'algue OSF sur la perméabilité paracellulaire in vivo

### i. En situation basale

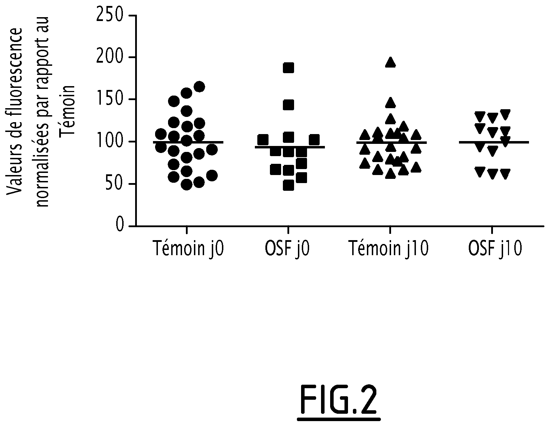

Au jour 0, la perméabilité cellulaire in vivo était identique chez les souris prédestinées à être gavées avec l'extrait OSF (n=12) et les souris témoins (n=24) (T test, Mann et Whitney test, p > 0.05) (Figure 2).

Après 10 jours de gavage, la perméabilité paracellulaire in vivo restait identique entre les souris OSF (n=12) et les souris témoins (n=24) (T test, Mann et Whitnez test, p > 0.05) (Figure 2).

### ii. En situation de stress

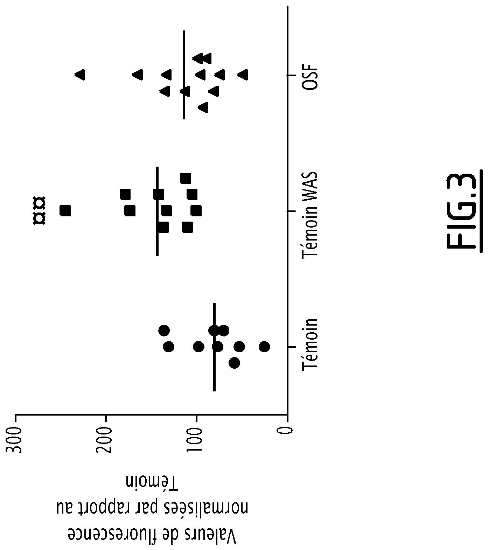

Le modèle de stress (Water Avoidance Stress (WAS)) induisait une augmentation de perméabilité paracellulaire. Les souris témoins WAS (témoins et OSF WAS) avaient une perméabilité plus élevée que les souris non stressées (n=9) gavées à l'eau (Témoin / OSF WAS, T test, Mann et Whitney test, p = 0.0949 ; Témoin / Témoin WAS, T test, Mann et Whitney test, p = 0.0021¤) (Figure 3).

La perméabilité paracellulaire in vivo avait tendance à être réduite chez les souris OSF WAS (n=12) par rapport aux souris témoin WAS (n=10) (T test, Mann et Whitney, p = 0.0602) (Figure 3).

### Effet de l'extrait d'algue OSF sur la motricité digestive

### i. En situation basale

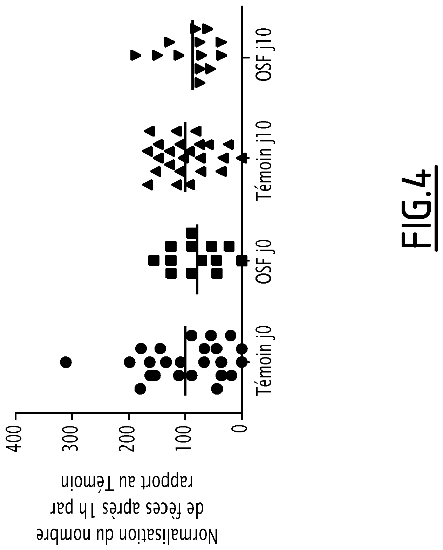

Le temps de transit total était identique chez les souris OSF et les souris témoins, en situation basale, du jour 0 au jour 10 (Figure 4).

Au jour 0 et après 10 jours de gavage, le transit colique était identique chez les souris du groupe OSF (n=12) par rapport aux souris du groupe témoin (n=24) (T test, Mann et Whitney test, p > 0.05) (Figure 4).

### ii. En situation de stress

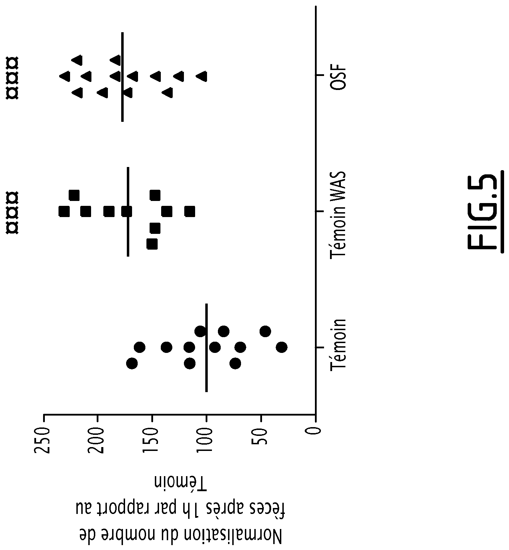

Le transit colique était identique chez les souris OSF WAS (n=13) par rapport aux souris témoin WAS (n=12) (T test, Mann et Whitney test, p>0.05) (Figure 5).

Le transit colique était significativement augmenté chez les souris témoins WAS et OSF WAS par rapport aux souris témoins (n=12) (T test, Mann et Whitney test; Témoin/Témoin WAS, p = 0.0019¤ ; témoin/OSF WAS, p = 0.0005¤) (Figure 5).

### Effet de l'extrait d'algue OSF sur la morphométrie/anatomie

### i. Poids des animaux

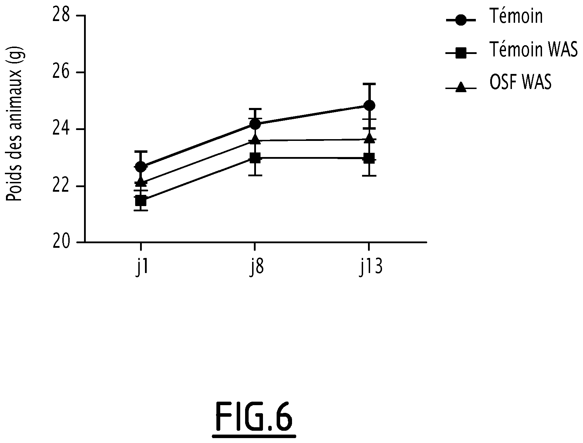

Chez les souris témoins (n=6), le poids augmentait significativement au cours du temps (One way anova, Friedman test, p = 0.0001*). Chez les souris traitées avec l'extrait d'algue OSF (n=6), le poids augmentait significativement au cours du temps (One way anova, Friedman test, p = 0.0001*). Chez les souris témoins WAS (n=6), le poids augmentait significativement au cours du temps (One way anova, Friedman test, p=0.0017*) (Figure 6).

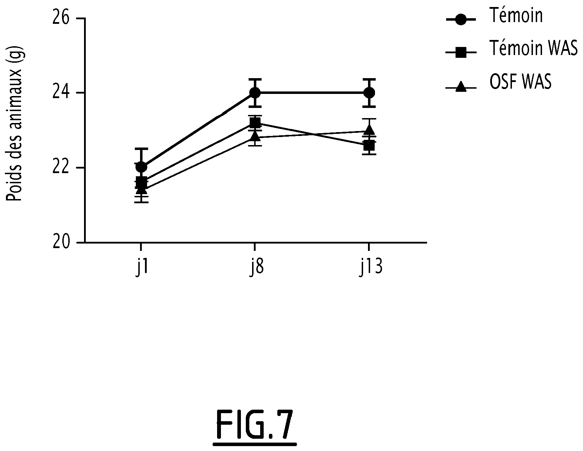

Chez les souris témoin (n=6), le poids augmentait significativement au cours du temps (One way anova, Friedman test, p =0.0001*). Chez les souris traitées avec l'extrait d'algue OSF (n=7), le poids augmentait significativement au cours du temps (One way anova, Friedman test, p < 0.0001*). Chez les souris témoins WAS (n=5), le poids augmentait significativement au cours du temps (One way anova, Friedman test, p = 0.0008*) (Figure 7).

### ii. Longueur de l'intestin

La longueur totale de l'intestin, ainsi que la longueur totale du côlon, étaient identiques chez les souris témoin, témoin WAS et les souris OSF WAS (One way anova, Kruskal-Wallis test, p>0.05).

### iii. Poids du cerveau

Le poids du cerveau avait tendance à être augmenté chez les souris OSF (n=13) par rapport aux souris témoin (n=24) (T test, Mann et Whitney test, p = 0.0579).

### Récapitulatif des données et données comparatives

L'impact de six extraits d'algues a été évalué sur les fonctions clefs du tube digestif : la perméabilité et la motricité intestinale, dans un modèle murin *in vivo.* Cette étude était réalisée en comparant les réponses induites par ces 6 différents extraits à un groupe contrôle gavé par l'excipient, dans des conditions de base et dans des conditions de stress d'évitement de l'eau (WAS ; Water Avoidance Stress).

Le Tableau 4 ci-dessous récapitule les données obtenues pour chacun des extraits.

**Tableau 4 : Synthèse de l'effet de six extraits d'algues sur la perméabilité et la motricité intestinale, dans un modèle murin**

| **Extrait** | **Perméabilité paracellulaire *in vivo*** | | **Temps transit total** | **Transit colique in vivo** | | **Poids des animaux** | **Longueur intestin /côlon** | **Poids du cerveau** |
|---|---|---|---|---|---|---|---|---|
| | **Basal** | **stress** | **basal** | **basal** | **stress** | | | |
| OSA | J0 = | ↑après stress | J0 = | J0 = | ↑ | "= ou ↑ " | pas d'effet | pas d'effet |
| | J10↓ | | J10 = | J10 = | | que témoin | | |
| OSB | J0 = | ↑après stress | J0 = | J0 = | ↑ | "= ou + " | pas d'effet | ↑ |
| | J10↓ | | J10 = | J10 = | | que témoin | | |
| OSC | | ↑après stress | | J0 ↑ | ↑ | "= " témoin | pas d'effet | pas d'effet |
| | J0 ↑ J10 = | | J10 J0↓ = | J10 = | | | | |
| OSD | J0 = | ↑après stress | J0 = | J0 = | ↑ | ↓ que témoin | pas d'effet | pas d'effet |
| | J10 ↑ | | J10 = | J10 = | | | | |
| **OSE** | **J0** = | **"=** " **sans stress** | **J0 =** | **J0 =** | **"=** " **sans stress** | **"=** " **témoin** | **pas d'effet** | **pas d'effet** |
| | **J10** = | | **J10 =** | **J10 =** | | | | |
| **OSF** | **J0 =** | **"= " sans stress** | J0 = | J0 = | ↑ | **"=** " **témoin** | **pas d'effet** | ↑ (tendance) |
| | **J10 =** | | J10 = | J10 = | | | | |

Les extraits d'algues OSE et OSF selon l'invention se distinguent des quatre autres extraits d'algues testés.

Cette étude, a montré que le complément OSF avait une forte tendance à diminuer la perméabilité en condition de stress (Water Avoidance Stress). Cet extrait n'induisait pas de modifications morpho-anatomiques, aucune différence n'était observée concernant l'évolution du poids des animaux, la longueur totale de l'intestin et le poids des cerveaux. Les taux de corticostérone, dans le sang, étaient identiques entre les souris témoin WAS et les souris gavées avec l'extrait d'algue OSF WAS.

### Exemple 4: Etude des effets de l'extrait d'algues OSE sur la motricité et la perméabilité intestinale

Cette étude est la synthèse de deux études indépendantes de caractérisation des effets de l'extrait OSE (l'oligosaccharide « E ») sur les fonctions digestives *in vivo* (perméabilité, motricité), et *ex vivo* sur la fonction de barrière intestinale en chambre d'Ussing. La capacité d'OSE à modifier ces fonctions digestives à la fois à leur état de base et lors d'un modèle de stress (Stress d'évitement de l'eau - ST), connu pour les modifier, a été évaluée. A la fin de cette phase de stress, l'impact d'OSE sur la réponse au stress était également évalué en mesurant le taux sanguin de corticostérone (hormone du stress).

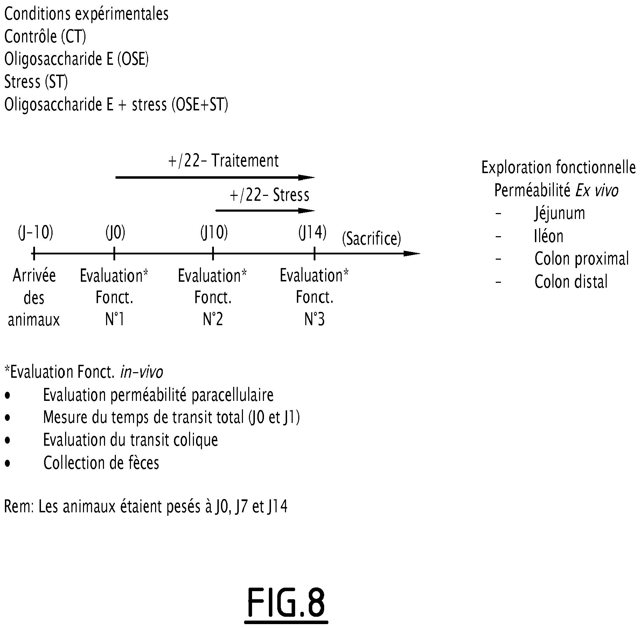

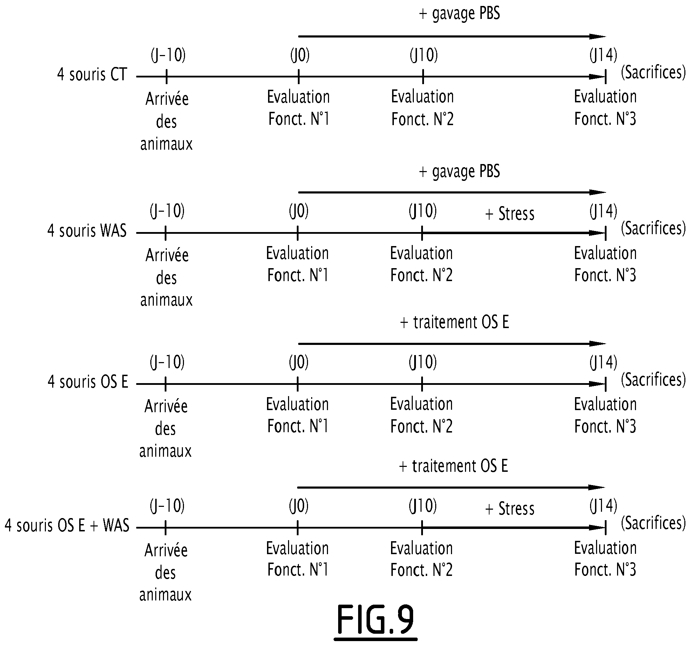

Le protocole général de l'étude ainsi que les détails de chaque série d'expériences sont montrées en figures 8 et 9.

### MATERIELS ET METHODES

### Modèle animal

Le modèle animal utilisé était la souris saine C57 black 6Rj mâle âgée de 8 semaines. Après 10 jours d'acclimatation, les souris ont été gavées quotidiennement (week-ends exclus) durant 14 jours avec l'oligosaccharide E (OSE) ou bien un placebo (eau). A partir du 11^{ème} jour de traitement, les souris étaient placées durant 1 heure sur un plot entouré d'eau durant 4 jours consécutifs jusqu'au 14^{ème} jour de traitement.

Les souris étaient hébergées dans des cages sur portoir ventilé. Au total 64 souris ont été utilisées avec 4 groupes expérimentaux distincts : un groupe de 28 souris contrôle gavées à l'eau, sans stress (groupe CT) ; un groupe de 28 souris contrôles gavées à l'eau, avec stress (groupe ST) ; un groupe de 16 souris gavées avec OSE (groupe OSE), un groupe de 28 souris gavées avec OSE, avec stress (OSE+ST). L'ensemble de l'étude s'est déroulée sur 6 séries d'expériences réalisées à des temps différents. Chaque série d'expériences était composée des quatre groupes (CT, ST, OSE et OSE+ST).

### A. Evaluation des effets d'OSE sur la perméabilité paracellulaire et transcellulaire in vivo et ex vivo.

### Evaluation de la perméabilité intestinale in vivo

Les perméabilités paracellulaire et transcellulaire étaient évaluées *in vivo* avant le début des gavages (J0) puis après 10 jours (J10) et 14 jours (J14) de gavages en mesurant le passage sanguin d'acide sulfonique conjugué au FITC (ASF - marqueur de faible poids moléculaire, 400 Da) et de la protéine Horse Radish Peroxydase (HRP - marqueur de haut poids moléculaire, 44kDa), respectivement.

Les souris étaient gavées avec une solution de carboxy-méthyl-cellulose 0.5% (p/v) contenant l'ASF (10 mg/ml) et la HRP (10 mg/mL). Le sang était collecté 120 min après le gavage puis le plasma était obtenu après une centrifugation à 3000g/5min. L'intensité de fluorescence du plasma était mesurée à l'aide d'un lecteur de plaque fluorimètre. La perméabilité à l'ASF est exprimée en pourcentage par rapport la moyenne de l'intensité de fluorescence du groupe CT. La perméabilité à I'HRP est évaluée en mesurant son activité enzymatique dans le plasma à l'aide d'un dosage enzymatique colorimétrique. La perméabilité à I'HRP est exprimée en pourcentage par rapport la moyenne des activités enzymatiques du groupe CT.

### Evaluation de la perméabilité intestinale ex vivo

Les perméabilités paracellulaire et transcellulaire étaient évaluées *ex vivo* à J14 sur le jéjunum, l'iléon, le colon proximal et le colon distal. Brièvement, les segments d'intestin étaient ouverts le long de l'insertion du mésentère et montés dans les chambres d'Ussing. Après une période d'équilibration de 30 min, ASF (1 mg/ml) et I'HRP (3.75 mg/ml) étaient ajoutés au pôle apical du tissu. La perméabilité à l'ASF était évaluée en mesurant la fluorescence d'échantillons de milieu prélevés toutes les 30 min au pôle basolatéral sur une période de 150 min. la quantification de fluorescence au cours du temps permet de réaliser une régression linéaire donc la pente définit le flux d'ASF au travers du tissu. La perméabilité à I'HRP était évaluée en mesurant son activité enzymatique dans des échantillons de milieu prélevés toutes les 30 min au pôle basolatéral sur une période de 150 min (dosage enzymatique colorimétrique).

### B. Evaluation des effets d'OSE sur la motricité intestinale in vivo.

### Evaluation du temps de transit total :

Le temps de transit total a été évalué à J0 et J10. La solution utilisée pour évaluer la perméabilité *in vivo* contenait également du rouge carmin (60mg/ml) qui n'était pas absorbé et était utilisé pour caractériser le temps de transit intestinal oro-anal (Tasselli *et al,* 2013). Le temps de transit total était déterminé en mesurant le temps écoulé entre le gavage et l'émission de la première fèces de couleur rouge.

### Evaluation du transit colique :

Le transit colique a été évalué à J0, J10, J14 en dénombrant le nombre de fèces émises durant 2 heures (Tasselli *et al,* 2013). Chaque souris était placée en cage individuelle et les fèces ont été comptées et collectées pendant 2h. Les fèces ont été pesées (poids frais) puis séchées 48 heures à 56°C et de nouveau repesées pour déterminer le pourcentage d'humidité (J0 et J10).

Le temps de transit total et le transit colique sont présentés en % par rapport à la moyenne des contrôles (% / moy. CT)

### C. Prélèvement et stockage d'échantillons

Au sacrifice (J14), des segments de jéjunum, d'iléon, de colon proximal et de colon distal ont été prélevés et congelés (-80°C) dans des tubes secs, et d'autre part, fixés au PBS/paraformaldéhyde 4%. Le cerveau était également prélevé, la moitié était congelée à -80°C et l'autre fixée. Ces échantillons pourront être utilisés pour d'éventuelles analyses transcriptomiques (tubes secs) et histologiques (tissus fixés). Des fèces étaient également prélevées à J0 et J10 puis congelées à -80°C.

### D. Etude de l'impact d'OSE sur la réponse au stress.

L'impact d'OSE sur le stress éprouvé par l'animal était évalué en mesurant le taux de corticostérone sanguin lors du stress d'évitement de l'eau. La concentration sanguine en corticostérone était déterminée par dosage ELISA et exprimée en ng/ml de plasma.

### Graphiques et Analyses statistiques :

Tous les graphiques et les évaluations statistiques étaient réalisés avec GraphPad Prism Software (GraphPad Software, Inc., La Jolla, California, United States). L'effet du traitement avec OSE au cours du temps était évalué avec une anova à 2 voies à mesure répétée suivi d'un post test de Bonferroni. Les effets d'OSE et du stress étaient évalués avec une anova à 2 voies à mesure répétée suivi d'un post test de Bonferroni (symbole : *). Le test de Mann et Whitney (symbole : ^{Φ})était utilisé pour comparer les données non appariées et déterminer la valeur exacte de p. Les valeurs de p ≤ 0,05 étaient considérées comme statistiquement significatives. Enfin, quand la probabilité (p) entre deux groupes donnés était comprise entre 0,07 <p> 0,05 , cette tendance était symbolisée par ^{Ω}.

### RESULTATS

### Effet d'OSE sur les paramètres anatomique

L'impact de 10 jours et 14 jours d'administration d'OSE était évalué sur plusieurs paramètres anatomiques tels que le poids et la longueur de l'intestin.

### i. Effet sur le poids des souris

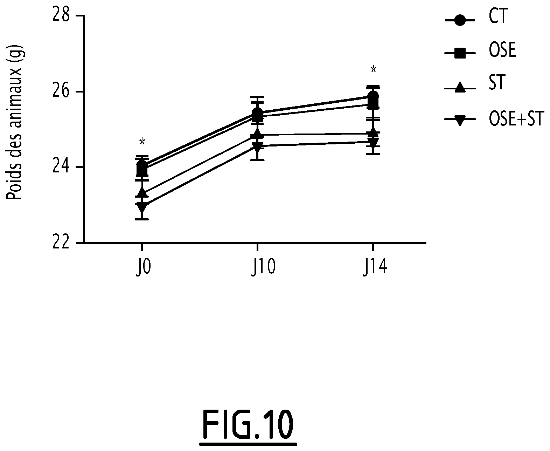

Entre J0 et J14, le poids des souris augmentait significativement dans les différents groupes (one way anova RM ; Friedman test p ≤ 0.0004 ; post test de Dunn's p < 0,01 ; n 28 CT ; 15 OSE ; 28 ST 27 OSE+ST). Après 10 jours de traitement le poids des souris était semblable entre les groupes, cependant à J14 le poids des souris OSE+ST est significativement plus faible que celui du groupe CT (Anova à 2 voies à mesure répétée ; p = 0,13 ; n = 24 / groupe ; Figure 10).

### ii. Effet sur la longueur totale de l'intestin

Les longueurs totales des intestins étaient similaires entre les groupes (Anova à 2 ; effet traitement p = 0,43 ; effet du stress p = 0,5 ; n = 28CT ; 15 OSE ; 28 ST et 27 OSE+ST) (données non montrées).

### iii. Effet sur la longueur de l'intestin grêle

La longueur de l'intestin grêle n'étais pas modifiée par le stress et / ou le traitement avec OSE (Anova à 2 voies ; effet traitement p = 0,51 ; effet du stress p = 0,1 ; n = 28 CT ; 15 OSE ; 28 ST et 27 OSE+ST) (données non montrées).

### iv. Effet sur la longueur du colon

La longueur colon n'étais pas modifiée par le stress et / ou le traitement avec OSE (Anova à 2 voies ; effet traitement p = 0,057; effet du stress p = 0,084 ; n = 28 CT ; 15 OSE ; 28 ST et 27 OSE+ST) (données non montrées).

### v. Effet sur le poids du cerveau

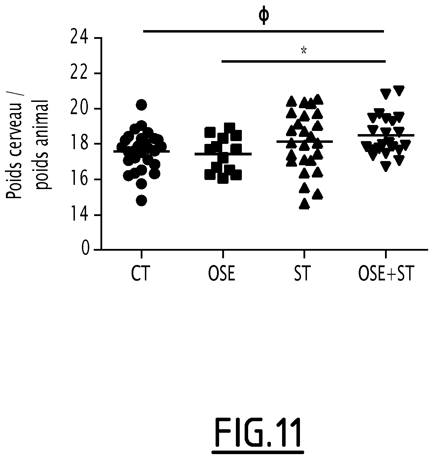

Le stress induisait des modifications significatives du poids des cerveaux (Anova à deux voies, traitement p = 0.85 ; stress p = 0,0039 ; n = 28 CT, 13 OSE, 26 ST, 22 OSE+ST). Le stress induisait une augmentation significative du poids des cerveaux du groupe OSE+ST par rapport aux groupes OSE (post test de Bonferroni p < 0,05*) et CT (Mann et Whitney p = 0,019^{Φ}) (Figure 11).

### Effet d'OSE sur la perméabilité in vivo à l'ASF

### i. En condition normale (J0 à J10)

Apres 10 jours de traitement, la perméabilité à l'ASF était semblable entre les groupes et entre J0 et J10 (Anova à 2 voies à mesure répétée ; traitement p = 0,26 ; temps p = 0,94 ; n = 44CT et 43 OSE) (données non montrées).

### ii. En condition de stress (J14)

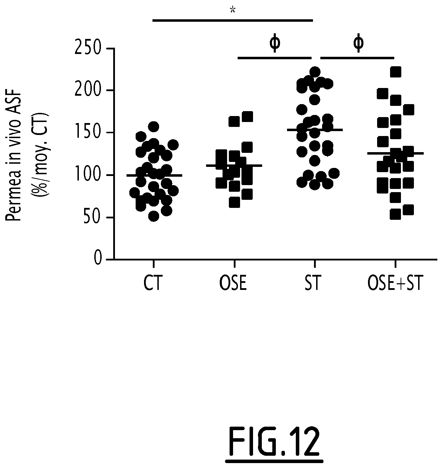

Le stress induisait une modification significative de la perméabilité *in vivo* à l'ASF (Anova à 2 voies; stress p < 0,0001 ; traitement p = 0,33). La perméabilité *in vivo* à l'ASF était significativement augmentée dans le groupes ST comparés aux groupes CT, OSE et OSE+ST (respectivement post-test de Bonferroni p < 0.001 * ; test de Mann et Whitney p = 0,0036^{Φ}; test de Mann et Whitney p = 0.038^{Φ}). La perméabilité *in vivo* à l'ASF était semblable entre le groupe OSE et le groupe OSE+ST (n = 27 CT, 15 OSE, 27 ST et 22 OSE+ST) (Figure 12).

### Effet d'OSE sur la perméabilité ex vivo à l'ASF

### i. Jéjunum

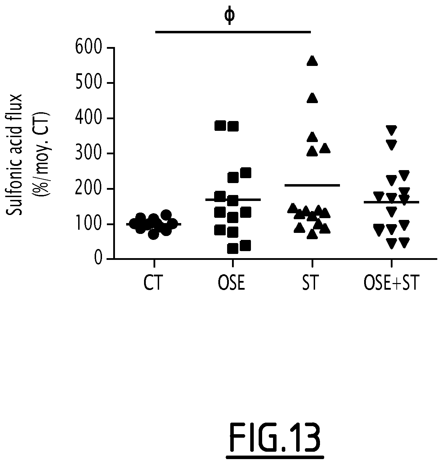

Une analyse statistique globale de l'effet du stress et/ou du traitement ne révélait pas de changement significatif de la perméabilité à l'ASF au niveau du jéjunum (Anova à 2 voies; stress p = 0,16 ; traitement p = 0,95 ; n = 12 CT, 13 OSE, 15 ST et 15 OSE+ST). Une analyse spécifique entre deux groupes données indiquait une augmentation significative de la perméabilité à l'ASF dans le groupe ST comparé au groupe CT (test de Mann et Whitney p = 0.0097^{Φ}) (Figure 13).

### ii. Iléon

L'analyse statistique globale révélait une forte tendance du stress à modifier la perméabilité à l'ASF au niveau de l'iléon entre les différents groupes (Anova à 2 voies; stress p = 0,05 ; traitement p = 0,79 ; n = 15 CT, 12 OSE, 12 ST et 12 OSE+ST). Une analyse spécifique entre les groupes n'indiquait pas de variation significative entre deux groupes donnés (Mann et Whitney) (données non montrées).

### iii. Colon proximal

Le stress et / ou le traitement n'induisaient pas de changement significatif de la perméabilité *ex vivo* à l'ASF au niveau du colon proximal (Anova à 2 voies; stress p = 0,17; traitement p = 0,92 ; n = 15 CT, 12 OSE, 12 ST et 12 OSE+ST) (données non montrées).

### iv. Colon distal

L'analyse statistique globale indiquait que le stress induisait une modification significative de la perméabilité à l'ASF au niveau du colon distal (Anova à 2 voies; stress p = 0,034 ; traitement p = 0,13). Une analyse spécifique entre les groupes ne révélait pas de variation significative entre deux groupes donnés (Mann et Whitney ; n = 14 CT, 15 OSE, 15 ST et 13 OSE+ST) (données non montrées).

### Effet d'OSE sur la perméabilité in vivo à I'HRP

### i. En condition normale (J0 à J10)

Apres 10 jours de traitement, la perméabilité à I'HRP était semblable entre les groupes et entre J0 et J10 (Anova à 2 voies à mesure répétée ; traitement p = 0,85 ; temps p = 0,45 ; n = 32 CT et 31 OSE) (données non montrées).

### ii. En condition de stress (J14)

Le stress et / ou le traitement n'induisaient pas de changement significatif de la perméabilité *in vivo* à la HRP (Anova à 2 voies; stress p = 0.75 ; traitement p = 0,7 ; n = 16 CT, 14 OSE, 15 ST et 16 OSE+ST) (données non montrées).

### Effet d'OSE sur la perméabilité ex vivo à I'HRP

### i. Jéjunum :

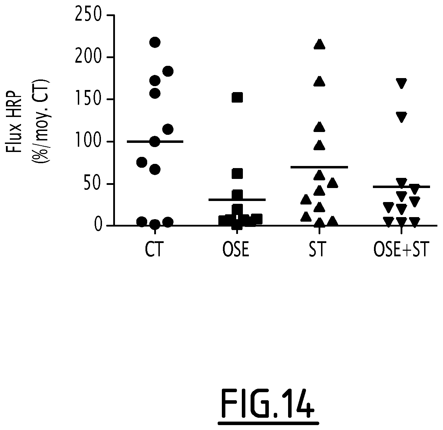

L'analyse statistique global indiquait que le traitement induisait une modification significative de la perméabilité à I'HRP au niveau du jéjunum (Anova à 2 voies ; traitement p = 0,02 ; stress p = 0,7). Une analyse spécifique entre les groupes ne révélait pas de variation significative entre deux groupes donnés (Mann et Whitney ; n = 11 CT, 10 OSE, 12 ST et 11 OSE+ST) (Figure 14).

### ii. Iléon

Le stress et / ou le traitement n'induisaient pas de changement significatif de la perméabilité à I'HRP au niveau de l'iléon entre les différents groupes (Anova à 2 voies; stress p = 0,17 ; traitement p = 0,99 ; n = 11 CT, 8 OSE, 11 ST et 8 OSE+ST) (données non montrées).

### iii. Colon proximal

Le stress et / ou le traitement n'induisaient pas de changement significatif de la perméabilité à I'HRP au niveau du colon proximal entre les différents groupes (Anova à 2 voies ; traitement p = 0,43 ; stress p = 0,71 ; n = 13 CT, 11 OSE, 10 ST et 12 OSE+ST) (données non montrées).

### iv. Colon distal

Le stress et / ou le traitement n'induisaient pas de changement significatif de la perméabilité à I'HRP au niveau du colon distal entre les différents groupes (Anova à 2 voies ; traitement p = 0,99 ; stress p = 0,15 ; n = 9 CT, 8 OSE, 8 ST et 5 OSE+ST) (données non montrées).

### Effet d'OSE sur la motricité intestinale

### i. Temps de transit total en condition normale J0 ― J10 (transit oro-anal)

Après 10 jours de traitement, le temps de transit total était semblable entre les 2 groupes et pas significativement différent entre J0 et J10 au sein de chaque groupe (Anova à 2 voies à mesures répétées ; traitement p = 0,58 ; temps p = 0,26 ; n = 48 CT et 34 OSE) (données non montrées).

### ii. Transit colique en condition normale (JO-J10)

Après 10 jours de traitement, le transit colique était semblable entre les 2 groupes et pas significativement différent entre J0 et J10 au sein de chaque groupe (Anova à 2 voies à mesures répétées ; traitement p = 0,99 ; temps p = 0,42 ; n = 44CT et 38 OSE) (données non montrées).

### iii. Transit colique en condition de stress (J14)

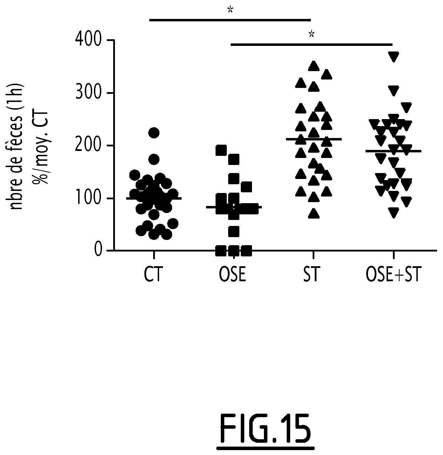

Le stress induisait une augmentation significative du nombre de fèces émises dans les groupes ST et OSE+ST comparés respectivement aux groupes CT et OS (Anova à 2 voies; stress p < 0.0001 ; traitement p = 0,13 ; post de test de Bonferroni P < 0.001* ; n = 28 CT, 16 OSE, 28 ST et 27 OSE+ST) (Figure 15).

### Caractérisation des fèces : poids frais / poids sec et % d'humidité (J0-J10)

### i. Fèces : poids frais

Après 10 jours de traitement, le poids frais moyen des fèces était semblable entre les 2 groupes et pas significativement différent entre J0 et J10 au sein de chaque groupe (Anova à 2 voies à mesures répétées ; traitement p = 0,31 ; temps p = 0,14 ; n = 32 CT et 30 OSE) (données non montrées).

### ii. Fèces : poids sec

Après 10 jours de traitement, le poids sec moyen des fèces était semblable entre les 2 groupes et pas significativement différent entre J0 et J10 au sein de chaque groupe (Anova à 2 voies à mesures répétées ; traitement p = 0,83 ; temps p = 0,046 ; n = 32 CT et 30 OSE) (données non montrées).

### iii. Fèces : % humidité

Après 10 jours de traitement, le pourcentage d'humidité des fèces était semblable entre les 2 groupes et pas significativement différent entre J0 et J10 au sein de chaque groupe (Anova à 2 voies à mesures répétées ; traitement p = 0,43 ; temps p = 0,56 ; n = 32 CT et 30 OSE) (données non montrées).

### Effet d'OSE sur la sécrétion de corticostérone induite par le stress

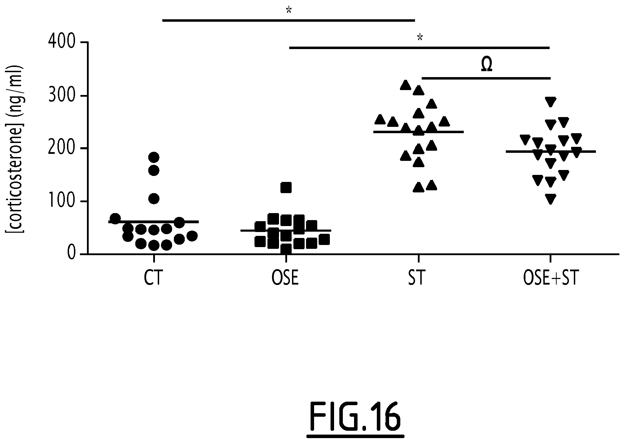

Le stress et le traitement induisaient une modification significative du taux de corticostérone sanguin (Anova à 2 voies; stress p < 0,0001 ; traitement p = 0,031 ; n = 15 CT, 15 OSE, 16 ST et 16 OSE+ST. Le taux de corticostérone augmentait significativement dans les groupes ST et OSE+ST comparés respectivement aux groupes CT et OSE (post de test de Bonferroni P < 0.001*). Une analyse statistique spécifique entre les groupes ST et OSE+ST indiquait que le taux de corticostérone tendait fortement à être inférieur dans le groupe OSE+ST (Mann et Whitney p = 0,068^{Ω}) (Figure 16).

**Tableau 5 : résumé des données de deux études des effets de l'extrait d'algue OSE**

| | **paramètres** | **J0** - **J10** | **J14 (-/+ST)** |
|---|---|---|---|
| **Données anatomiques** | Poids souris | CT = OSE | CT = OSE = ST ; OSE+ST < CT* |
| | Longueur totale intestin | | CT = OSE = ST = OSE+ST |
| | Longueur intestin grêle | | CT = OSE = ST = OSE+ST |
| | Longueur colon | | CT = OSE = ST = OSE+ST |
| **Perméabilité à l'ASF** | *In vivo* | CT = OSE | CT < ST* ; OSE < OSE+ST^{Φ} ; OSE+ST < ST^{Φ} |
| | *Ex vivo* jéjunum | | CT < ST^{Φ} |
| | *Ex vivo* iléon | | CT = OSE = ST = OSE+ST |
| | *Ex vivo* colon distal | | CT = OSE = ST = OSE+ST |
| **Perméabilité à I'HRP** | *In vivo* | CT = OSE | CT = OSE = ST = OSE+ST |
| | *Ex vivo* jéjunum | | CT = OSE = ST = OSE+ST |
| | *Ex vivo* iléon | | CT = OSE = ST = OSE+ST |
| | *Ex vivo* colon distal | | CT = OSE = ST = OSE+ST |
| **Motricité intestinale** | Temps de transit total | CT = OSE | |
| | Transit colique (FPO) | CT = OSE | CT < ST* ; OSE < OSE+ST* ; ST = OSE+ST |
| | Poids moyen fèces fraiches | CT = OSE | |
| | Poids moyen fèces sèches | CT = OSE | |
| | % humidité fèces | CT = OSE | |
| **[Corticostérone]** | Effet du stress (ST) | | CT < ST* ; OSE < OSE+ST* ; OSE+ST< ST^{Ω} |

### Conclusion

Les résultats obtenus avec OSE dans l'étude complémentaire permettent de confirmer les effets observés lors de l'étude préliminaire, et la synthèse des résultats de ces deux études aboutit aux conclusions suivantes :
En conditions basales, le complément OSE ne modifiait pas les perméabilités paracellulaire (ASF) et transcellulaire (HRP) *in vivo* et *ex vivo* ainsi que la motricité globale et colique *in vivo.* De plus, les principaux paramètres anatomiques mesurés
(poids souris, longueur grêle, colon) ainsi que la caractérisation des fèces présentaient des valeurs semblables entre le groupe OSE et CT. Enfin, les taux de corticostérone plasmatique étaient similaires dans les groupes CT et OSE.
En conditions de stress, la perméabilité paracellulaire *in vivo* était augmentée significativement dans les groupes ST et OSE+ST comparés respectivement aux groupes CT et OSE. Cependant, cette augmentation était moins importante dans le groupe OSE+ST et de plus, la perméabilité paracellulaire du groupe OSE était significativement inférieure à celle du groupe ST. Le complément OSE limite donc significativement l'augmentation de perméabilité paracellulaire *in vivo* induite par le stress. Cette augmentation de la perméabilité paracellulaire par le stress a été également mise en évidence *ex vivo* au niveau du jéjunum. Cependant, il n'y avait pas de différence statistique entre les groupes ST et OSE+ST, ceci est probablement dû à la dispersion des points au sein de ces deux groupes.

Enfin, le stress induisait une augmentation significative du taux de corticostérone plasmatique dans le groupe ST comparé au groupe CT. Cependant, bien que le taux de corticostérone soit aussi augmenté dans OSE+ST par rapport à OSE, ce taux tend fortement (p = 0.068) à être diminué dans le groupe OSE+ST par rapport au groupe ST, suggérant qu'OSE limite l'effet du stress.

En conclusion, cette seconde étude a permis de confirmer les résultats préliminaires obtenus. La synthèse de ces deux études renforce encore l'intérêt du composé OSE, notamment ses propriétés vis-à-vis de la réponse au stress.

## Revendications

1. Extrait d'algues rouges de l'espèce *Solieria chordalis* pour son utilisation pour la réduction ou la prévention d'un trouble intestinal, **caractérisé en ce que** l'extrait d'algues rouges de l'espèce *Solieria chordalis* protège et/ou améliore la fonction ou l'intégrité de la barrière épithéliale intestinale, et **en ce que** l'extrait d'algues rouges est obtenu par un procédé de préparation comprenant :
a) le lavage et dessablage des algues;
b) le broyage des algues lavées et dessablées ;
c) l'hydrolyse thermique du broyat obtenu;
d) la séparation de la phase solide du broyat de sa phase liquide ;
e) la clarification de ladite phase liquide ;
f) l'ultrafiltration du jus de filtration obtenu à l'étape e) sur une membrane ayant un seuil de coupure de 300 kDa; et
g) la récolte et le séchage du rétentat d'ultrafiltration obtenu à l'étape f) ;
ou
g) la récolte et le séchage du perméat d'ultrafiltration obtenu à l'étape f). **caractérisé en ce que** le trouble intestinal est une diarrhée, une constipation, un syndrôme du côlon irritable, ou une maladie inflammatoire chronique de l'intestin,
**caractérisé en ce que** l'extrait d'algues rouges de l'espèce *Solieria chordalis* réduit l'augmentation de la perméabilité intestinale et/ou du temps de transit intestinal,
et le trouble intestinal est induit par un stress.

2. Extrait d'algues rouges de l'espèce *Solieria chordalis* pour l'utilisation selon la revendication 1; **caractérisé en ce que** le procédé de préparation comprend en étape g) :
g) la récolte et le séchage du rétentat d'ultrafiltration obtenu à l'étape f).

3. Extrait d'algues rouges de l'espèce *Solieria chordalis* pour l'utilisation selon l'une quelconque des revendications 1-2; **caractérisé en ce que** le procédé de préparation comprend en étape g) :
g) la récolte et le séchage du perméat d'ultrafiltration obtenu à l'étape f).

4. Extrait d'algues rouges de l'espèce *Solieria chordalis* pour l'utilisation selon l'une quelconque des revendications 1 à 2, **caractérisé en ce que** le procédé de préparation comprend l'étape g) de récolte et séchage du rétentat d'ultrafiltration obtenu à l'étape f) et **en ce que** l'extrait d'algues rouges comprend (en poids sur le poids total de l'extrait) :
Matière sèche : 92-99% ;
Matière minérale : 5-37% ;
Sucres : 10-48% ;
Protéines : 10-30% ;
Sulfates : 10-20%.

5. Extrait d'algues rouges de l'espèce *Solieria chordalis* pour l'utilisation selon l'une quelconque des revendications 1 à 2 et 4, **caractérisé en ce que** le procédé de préparation comprend l'étape g) de récolte et séchage du rétentat d'ultrafiltration obtenu à l'étape f) et en ce et **en ce que** les monosaccharides constitutifs des polysaccharides de l'extrait d'algues rouges comprennent :
Galactose : 50-75%,
Glucose : 5-40% ; et
Xylose : 1-7%,
les pourcentages étant exprimés en ratio molaire des monosaccharides constitutifs des polysaccharides.

6. Extrait d'algues rouges de l'espèce *Solieria chordalis* pour l'utilisation selon l'une quelconque des revendications 1 à 2 et 4-5, **caractérisé en ce que** le procédé de préparation comprend l'étape g) de récolte et séchage du rétentat d'ultrafiltration obtenu à l'étape f) et en ce et **en ce que** au moins 40% de la matière organique de l'extrait d'algues rouges présente un poids moléculaire supérieur ou égal à 50 kDa, et de préférence au moins 40% de la matière organique de l'extrait d'algues rouges présente un poids moléculaire moyen supérieur ou égal à 250 kDa.

7. Extrait d'algues rouges de l'espèce *Solieria chordalis* pour l'utilisation selon l'une quelconque des revendications 1 et 3, **caractérisé en ce que** le procédé de préparation comprend l'étape g) de récolte et séchage du perméat d'ultrafiltration obtenu à l'étape f) et **en ce que** l'extrait d'algues rouges comprend (en poids sur le poids total de l'extrait) :
Matière sèche : 95-99% ;
Matière minérale : 5-65%;
Sucres : 5-37% ;
Protéines : 5-29% ;
Sulfates : 1-9%.

8. Extrait d'algues rouges de l'espèce *Solieria chordalis* pour l'utilisation selon l'une quelconque des revendications 1, 3 et 7, **caractérisé en ce que** le procédé de préparation comprend l'étape g) de récolte et séchage du perméat d'ultrafiltration obtenu à l'étape f) et **en ce que** les monosaccharides constitutifs des polysaccharides de l'extrait d'algues rouges comprennent :
Galactose : 60-85%,
Glucose : 10-25% ; et
Xylose : 1-5%,
les pourcentages étant exprimés en ratio molaire des monosaccharides constitutifs des polysaccharides.

9. Extrait d'algues rouges de l'espèce *Solieria chordalis* pour l'utilisation selon l'une quelconque des revendications 1, 3 et 7-8, **caractérisé en ce que** le procédé de préparation comprend l'étape g) de récolte et séchage du perméat d'ultrafiltration obtenu à l'étape f) et **en ce que** au moins au moins 70% de la matière organique de l'extrait d'algues rouges présente un poids moléculaire inférieur ou égal à 150 kDa, et de préférence au moins 70% de la matière organique de l'extrait d'algues rouges présente un poids moléculaire moyen inférieur ou égal à 50 kDa.

10. Extrait d'algues rouges de l'espèce *Solieria chordalis* susceptible d'être obtenu par un procédé de préparation comprenant :
a) le lavage et dessablage des algues ;
b) le broyage des algues lavées et dessablées ;
c) l'hydrolyse thermique du broyat obtenu ;
d) la séparation de la phase solide du broyat de sa phase liquide ;
e) la clarification de ladite phase liquide
f) l'ultrafiltration du jus de filtration obtenu à l'étape e) sur une membrane ayant un seuil de coupure de 300 kDa; et
g) la récolte et le séchage du rétentat d'ultrafiltration obtenu à l'étape f) ; ou
g) la récolte et le séchage du perméat d'ultrafiltration obtenu à l'étape f).

11. Extrait d'algues rouges de l'espèce *Solieria chordalis* selon la revendication 10, l'extrait d'algues rouges étant tel que défini dans l'une quelconque des revendications 4 à 6.

12. Extrait d'algues rouges de l'espèce *Solieria chordalis* selon la revendication 10, l'extrait d'algues rouges étant tel que défini dans l'une quelconque des revendications 7 à 9.

13. Extrait d'algues rouges de l'espèce *Solieria chordalis* tel que défini dans l'une quelconque des revendications 4 à 9.

14. Complément alimentaire, composition pharmaceutique ou un médicament comprenant un extrait d'algues rouges de l'espèce *Solieria chordalis* tel que défini dans l'une quelconque des revendications 4 à 13.

15. Procédé de préparation d'un extrait d'algues rouges de l'espèce *Solieria chordalis* comprenant :
a) le lavage et dessablage des algues;
b) le broyage des algues lavées et dessablées ;
c) l'hydrolyse thermique du broyat obtenu;
d) la séparation de la phase solide du broyat de sa phase liquide ;
e) la clarification de ladite phase liquide ;
f) l'ultrafiltration du jus de filtration obtenu à l'étape e) sur une membrane ayant un seuil de coupure de 300 kDa; et
g) la récolte et le séchage du rétentat d'ultrafiltration obtenu à l'étape f) ; ou
g) la récolte et le séchage du perméat d'ultrafiltration obtenu à l'étape f).

## Patentansprüche

1. Rotalgenextrakt der Spezies *Solieria chordalis* zur Verwendung für die Reduzierung oder Prävention einer Darmerkrankung, **dadurch gekennzeichnet, dass** der Rotalgenextrakt der Spezies *Solieria chordalis* die Funktion oder Unversehrtheit der Darmepithelbarriere schützt und/oder verbessert, und dass der Rotalgenextrakt durch ein Herstellungsverfahren erlangt wird, das Folgendes umfasst:
a) Waschen und Entsanden von Algen;
b) Zerkleinern von gewaschenen und entsandeten Algen;
c) thermische Hydrolyse des erlangten Mahlguts;
d) Trennen der festen Phase des Mahlguts von seiner flüssigen Phase;
e) Klären der flüssigen Phase;
f) Ultrafiltration des in Schritt e) erlangten Filtersafts auf einer Membran mit einem Trennungsschwellenwert von 300 kDa; und
g) Sammeln und Trocknen des in Schritt f) erlangten Ultrafiltrationsretentats; oder
g) Sammeln und Trocknen des in Schritt f) erlangten Ultrafiltrationspermeats. **dadurch gekennzeichnet, dass** die Darmstörung Durchfall, Verstopfung, Reizdarmsyndrom oder chronisch entzündliche Darmerkrankung ist,
**dadurch gekennzeichnet, dass** der Rotalgenextrakt der Spezies *Solieria chordalis* den Anstieg der Darmpermeabilität und/oder der Darmtransitzeit verringert,
und die Darmerkrankung durch Stress ausgelöst wird.

2. Rotalgenextrakt der Spezies *Solieria chordalis* zur Verwendung nach Anspruch 1, **dadurch gekennzeichnet, dass** das Herstellungsverfahren in Schritt g) Folgendes umfasst:
h) Sammeln und Trocknen des in Schritt f) erlangten Ultrafiltrationsretentats.

3. Rotalgenextrakt der Spezies *Solieria chordalis* zur Verwendung nach einem der Ansprüche 1-2, **dadurch gekennzeichnet, dass** das Herstellungsverfahren in Schritt g) Folgendes umfasst:
g) Sammeln und Trocknen des in Schritt f) erlangten Ultrafiltrationspermeats.

4. Rotalgenextrakt der Spezies *Solieria chordalis* zur Verwendung nach einem der Ansprüche 1 bis 2, **dadurch gekennzeichnet, dass** das Herstellungsverfahren den Schritt g) eines Sammelns und Trocknens des in Schritt f) erlangten Ultrafiltrationsretentats umfasst und dass der Rotalgenextrakt (bezogen auf das Gesamtgewicht des Extrakts) Folgendes umfasst:
Trockenmasse: 92-99 %;
Mineralstoffe: 5-37 %;
Zucker: 10-48 %;
Proteine: 10-30 %;
Sulfate: 10-20 %.

5. Rotalgenextrakt der Spezies *Solieria chordalis* zur Verwendung nach einem der Ansprüche 1 bis 2 und 4, **dadurch gekennzeichnet, dass** das Herstellungsverfahren den Schritt g) eines Sammelns und Trocknens des in Schritt f) erlangten Ultrafiltrationsretentats umfasst und dass die Monosaccharide, die Bestandteil der Polysaccharide des Rotalgenextrakts sind, Folgendes umfassen:
Galaktose: 50-75 %,
Glukose: 5-40 %; und
Xylose: 1-7 %,
wobei die Prozentsätze als Molverhältnis der Monosaccharide ausgedrückt sind, die Bestandteil der Polysaccharide sind.

6. Rotalgenextrakt der Spezies *Solieria chordalis* zur Verwendung nach einem der Ansprüche 1 bis 2 und 4 bis 5, **dadurch gekennzeichnet, dass** das Herstellungsverfahren den Schritt g) eines Sammelns und Trocknens des in Schritt f) erlangten Ultrafiltrationsretentats umfasst und dass mindestens 40 % der organischen Substanz des Rotalgenextrakts ein Molekulargewicht von größer als oder gleich wie 50 kDa aufweisen, und vorzugsweise mindestens 40 % der organischen Substanz des Rotalgenextrakts ein durchschnittliches Molekulargewicht größer als oder gleich wie 250 kDa aufweisen.

7. Rotalgenextrakt der Spezies *Solieria chordalis* zur Verwendung nach einem der Ansprüche 1 und 3, **dadurch gekennzeichnet, dass** das Herstellungsverfahren den Schritt g) eines Sammelns und Trocknens des in Schritt f) erlangten Ultrafiltrationspermeats umfasst und dass der Rotalgenextrakt (bezogen auf das Gesamtgewicht des Extrakts) Folgendes umfasst:
Trockenmasse: 95-99 %;
Mineralstoffe: 5-65 %;
Zucker: 5-37 %;
Proteine: 5-29 %;
Sulfate: 1-9 %.

8. Rotalgenextrakt der Spezies *Solieria chordalis* zur Verwendung nach einem der Ansprüche 1, 3 und 7, **dadurch gekennzeichnet, dass** das Herstellungsverfahren den Schritt g) eines Sammelns und Trocknens des in Schritt f) erlangten Ultrafiltrationspermeats umfasst und dass die Monosaccharide, die Bestandteil der Polysaccharide des Rotalgenextrakts sind, Folgendes umfassen:
Galaktose: 60-85 %,
Glukose: 10-25 %; und
Xylose: 1-5 %,
wobei die Prozentsätze als Molverhältnis der Monosaccharide ausgedrückt sind, die Bestandteil der Polysaccharide sind.

9. Rotalgenextrakt der Spezies *Solieria chordalis* zur Verwendung nach einem der Ansprüche 1, 3 und 7-8, **dadurch gekennzeichnet, dass** das Herstellungsverfahren den Schritt g) eines Sammelns und Trocknens des in Schritt f) erlangten Ultrafiltrationspermeats umfasst und dass mindestens mindestens 70 % der organischen Substanz des Rotalgenextrakts ein Molekulargewicht von kleiner als oder gleich wie 150 kDa aufweisen, und dass vorzugsweise mindestens 70 % der organischen Substanz des Rotalgenextrakts ein durchschnittliches Molekulargewicht kleiner als oder gleich wie 50 kDa aufweisen.

10. Rotalgenextrakt der Spezies *Solieria chordalis,* der durch ein Herstellungsverfahren erlangt werden kann, der Folgendes umfasst:
a) Waschen und Entsanden von Algen;
b) Zerkleinern von gewaschenen und entsandeten Algen;
c) thermische Hydrolyse des erlangten Mahlguts;
d) Trennen der festen Phase des Mahlguts von seiner flüssigen Phase;
e) Klären der flüssigen Phase;
f) Ultrafiltration des in Schritt e) erlangten Filtersafts auf einer Membran mit einem Trennungsschwellenwert von 300 kDa; und
g) Sammeln und Trocknen des in Schritt f) erlangten Ultrafiltrationsretentats; oder
g) Sammeln und Trocknen des in Schritt f) erlangten Ultrafiltrationspermeats.

11. Rotalgenextrakt der Spezies *Solieria chordalis* nach Anspruch 10, wobei der Rotalgenextrakt ist, wie es in einem der Ansprüche 4 bis 6 definiert ist.

12. Rotalgenextrakt der Spezies *Solieria chordalis* nach Anspruch 10, wobei der Rotalgenextrakt ist, wie es in einem der Ansprüche 7 bis 9 definiert ist.

13. Rotalgenextrakt der Spezies *Solieria chordalis,* wie er in einem der Ansprüche 4 bis 9 definiert ist.

14. Nahrungsergänzungsmittel, pharmazeutische Zusammensetzung oder Medikament, umfassend einen Rotalgenextrakt der Spezies *Solieria chordalis,* wie er in einem der Ansprüche 4 bis 13 definiert ist.

15. Verfahren zur Herstellung eines Rotalgenextrakts der Spezies *Solieria chordalis,* umfassend:
a) Waschen und Entsanden von Algen;
b) Zerkleinern von gewaschenen und entsandeten Algen;
c) thermische Hydrolyse des erlangten Mahlguts;
d) Trennen der festen Phase des Mahlguts von seiner flüssigen Phase;
e) Klären der flüssigen Phase;
f) Ultrafiltration des in Schritt e) erlangten Filtersafts auf einer Membran mit einem Trennungsschwellenwert von 300 kDa; und
g) Sammeln und Trocknen des in Schritt f) erlangten Ultrafiltrationsretentats; oder
g) Sammeln und Trocknen des in Schritt f) erlangten Ultrafiltrationspermeats.

## Claims

1. An extract of red seaweed of the species *Solieria chordalis* for use in the reduction or prevention of a bowel disorder, **characterised in that** the extract of red seaweed of the species *Solieria chordalis* protects and/or improves the function or integrity of the intestinal epithelial barrier, and **in that** the extract of red seaweed is obtained by a preparation method comprising:
a) washing and desanding the seaweed;
b) grinding the washed and desanded seaweed;
c) thermal hydrolysis of the resulting grind;
d) separating the solid phase of the grind from its liquid phase;
e) clarifying said liquid phase;
f) ultrafiltration of the filter juice obtained in step e) over a membrane with a cutoff threshold of 300 kDa; and
g) collecting and drying the ultrafiltration retentate obtained in step f); or
g) collecting and drying the ultrafiltration permeate obtained in step f). **characterised in that** the bowel disorder is diarrhoea, constipation, irritable bowel syndrome, or chronic inflammatory bowel disease,
**characterised in that** the extract of red seaweed of the species *Solieria chordalis* reduces the increase in intestinal permeability and/or intestinal transit time,
and the bowel disorder is stress-induced.

2. An extract of red seaweed of the species *Solieria chordalis* for use according to claim 1; **characterised in that** the preparation method comprises in step g):
h) collecting and drying the ultrafiltration retentate obtained in step f).

3. An extract of red seaweed of the species *Solieria chordalis* for use according to claims 1 or 2; **characterised in that** the preparation method comprises in step g):
g) collecting and drying the ultrafiltration permeate obtained in step f).

4. An extract of red seaweed of the species *Solieria chordalis* for use according to any one of claims 1 to 2, **characterized in that** the preparation method comprises step g) of collecting and drying the ultrafiltration retentate obtained in step f) and **in that** the red seaweed extract comprises (by weight relative to the total weight of the extract):
Dry matter: 92-99%;
Mineral matter: 5-37%;
Sugars: 10-48%;
Proteins: 10-30%;
Sulphates: 10-20%.

5. Extract of red seaweed of the species *Solieria chordalis* for use according to any one of claims 1 to 2 and 4, **characterised in that** the preparation method comprises step g) of collecting and drying the ultrafiltration retentate obtained in step f) and **in that** the constituent monosaccharides of the polysaccharides of the red seaweed extract comprise:
Galactose: 50-75%.
Glucose: 5-40%; and
Xylose: 1-7%.
the percentages being expressed in the molar ratio of the constituent monosaccharides of the polysaccharides.

6. Extract of red seaweed of the species *Solieria chordalis* for use according to any one of claims 1 to 2 and 4-5, **characterised in that** the preparation method comprises the step g) of collecting and drying the ultrafiltration retentate obtained in step f) and **in that** at least 40% of the organic matter of the red seaweed extract has a molecular weight greater than or equal to 50 kDa, and preferably at least 40% of the organic matter of the red seaweed extract has an average molecular weight greater than or equal to 250 kDa.

7. An extract of red seaweed of the species *Solieria chordalis* for use according to any one of claims 1 to 3, **characterised in that** the preparation method comprises step g) of collecting and drying the ultrafiltration retentate obtained in step f) and **in that** the extract of red seaweed comprises (by weight relative to the total weight of the extract):
Dry matter: 95-99%;
Mineral matter: 5-65%;
Sugars: 5-37%;
Proteins: 5-29%;
Sulphates: 1-9%.

8. Extract of red seaweed of the species *Solieria chordalis* for use according to any one of claims 1, 3 and 7, **characterised in that** the preparation method comprises step g) of collecting and drying the ultrafiltration permeate obtained in step f) and **in that** the constituent monosaccharides of the polysaccharides of the red seaweed extract comprise:
Galactose: 60-85%.
Glucose: 10-25%; and
Xylose: 1-5%.
the percentages being expressed in the molar ratio of the constituent monosaccharides of the polysaccharides.

9. Extract of red seaweed of the species *Solieria chordalis* for use according to any one of claims 1, 3 and 7-8, **characterised in that** the preparation method comprises the step g) of collecting and drying the ultrafiltration permeate obtained in step f) and **in that** at least 70% of the organic matter of the extract of red seaweed has a molecular weight less than or equal to 150 kDa, and preferably at least 70% of the organic matter of the extract of red seaweed has an average molecular weight less than or equal to 50 kDa.

10. Extract of red seaweed of the species *Solieria chordalis* obtainable by a preparation method comprising:
a) washing and desanding the seaweed;
b) grinding the washed and desanded seaweed;
c) thermal hydrolysis of the resulting grind;
d) separating the solid phase of the grind from its liquid phase;
e) clarifying said liquid phase;
f) ultrafiltration of the filter juice obtained in step e) over a membrane with a cutoff threshold of 300 kDa; and
g) collecting and drying the ultrafiltration retentate obtained in step f); or
g) collecting and drying the ultrafiltration permeate obtained in step f).

11. An extract of red seaweed of the species *Solieria chordalis* according to claim 10, the extract of red seaweed being as defined in any one of claims 4 to 6.

12. An extract of red seaweed of the species *Solieria chordalis* according to claim 10, the extract of red seaweed being as defined in any one of claims 7 to 9.

13. An extract of red seaweed of the species *Solieria chordalis* as defined in any of claims 4 to 9.

14. A dietary supplement, pharmaceutical composition or medication comprising an extract of red seaweed of the species *Solieria chordalis* as defined in any of claims 4 to 13.

15. A method for the preparation of an extract of red seaweed of the species *Solieria chordalis* comprising:
a) washing and desanding the seaweed;
b) grinding the washed and desanded seaweed;
c) thermal hydrolysis of the resulting grind;
d) separating the solid phase of the grind from its liquid phase;
e) clarifying said liquid phase;
f) ultrafiltration of the filter juice obtained in step e) over a membrane with a cutoff threshold of 300 kDa; and
g) collecting and drying the ultrafiltration retentate obtained in step f); or
g) collecting and drying the ultrafiltration permeate obtained in step f).
